# EUROPEAN PATENT SPECIFICATION

(11) **EP 2 781 598 B1**
(45) Date of publication and mention of the grant of the patent: **08.08.2018**
(21) Application number: 12850603.7
(22) Date of filing: 14.11.2012
(51) Int. Cl.: C12N 15/113, A61K 31/7105, A61K 31/713, A61P 35/00

(54) **NUCLEIC ACID MOLECULE FOR INHIBITING ACTIVITY OF RNAI MOLECULE**
NUKLEINSÄUREMOLEKÜL ZUR HEMMUNG DER AKTIVITÄT VON RNAI-MOLEKÜLEN
MOLÉCULE D'ACIDE NUCLÉIQUE POUR INHIBER L'ACTIVITÉ DE MOLÉCULE ARNI

(30) Priority: 16.11.2011 JP 2011250905
(43) Date of publication of application: 24.09.2014
(73) Proprietor: Osaka City University, Osaka-shi, Osaka 558-8585 (JP)
(72) Inventor: TACHIBANA, Akira, Osaka-shi, Osaka 558-8585 (JP); TANABE, Toshizumi, Osaka-shi, Osaka 558-8585 (JP)
(74) Representative: Denison, Christopher Marcus
(86) International application number: PCT/JP2012/079518
(87) International publication number: WO 2013/073576

(56) References cited:
- JP-A- 2003 500 064
- JP-A- 2010 509 923
- US-A- 5 514 546
- ANNALEEN VERMEULEN ET AL: "Double-stranded regions are essential design components of potent inhibitors of RISC function", RNA, COLD SPRING HARBOR LABORATORY PRESS, US, vol. 13, no. 5, 1 May 2007 (2007-05-01), pages 723-730, XP002659375, ISSN: 1355-8382, DOI: 10.1261/RNA.448107 [retrieved on 2007-03-30]
- ESAU ET AL: "Inhibition of microRNA with antisense oligonucleotides", METHODS, ACADEMIC PRESS, vol. 44, no. 1, 23 December 2007 (2007-12-23), pages 55-60, XP022398580, ISSN: 1046-2023, DOI: 10.1016/J.YMETH.2007.11.001
- K A LENNOX ET AL: "Chemical modification and design of anti-miRNA oligonucleotides", GENE THERAPY, vol. 18, no. 12, 14 July 2011 (2011-07-14) , pages 1111-1120, XP055156922, ISSN: 0969-7128, DOI: 10.1038/gt.2011.100
- KIM A LENNOX ET AL: "A Direct Comparison of Anti-microRNA Oligonucleotide Potency", PHARMACEUTICAL RESEARCH, KLUWER ACADEMIC PUBLISHERS-PLENUM PUBLISHERS, NL, vol. 27, no. 9, 28 April 2010 (2010-04-28) , pages 1788-1799, XP019827996, ISSN: 1573-904X
- BOUTLA A ET AL: "Developmental defects by antisense-mediated inactivation of micr-RNAs 2 and 13 in Drosophila and the identification of putative target genes", NUCLEIC ACIDS RESEARCH, OXFORD UNIVERSITY PRESS, GB, vol. 31, no. 17, 1 September 2003 (2003-09-01), pages 4973-4980, XP002993014, ISSN: 0305-1048, DOI: 10.1093/NAR/GKG707
- KHAN I. M. ET AL.: 'A novel method to stabilise antisense oligonucleotides against exonuclease degradation' NUCLEIC ACIDS RES vol. 21, no. 12, 1993, pages 2957 - 2958, XP055065596
- YOSHIZAWA S. ET AL.: 'Nuclease resistance of an extraordinarily thermostable mini-hairpin DNA fragment, d(GCGAAGC) and its application to in vitro protein synthesis' NUCLEIC ACIDS RES vol. 22, no. 12, 1994, pages 2217 - 2221, XP003031026
- ICHIRO HIRAO ET AL.: 'Unusual Single-Stranded DNA Structures: Extraordinarily Thermo-Stable Mini-Hairpin' PROTEIN, NUCLEIC ACID AND ENZYME vol. 40, no. 10, 1995, pages 1583 - 1591, XP008161350
- XIAO J. ET AL.: 'Novel approaches for gene- specific interference via manipulating actions of microRNAs: examination on the pacemaker channel genes HCN2 and HCN4' J CELL PHYSIOL vol. 212, 2007, pages 285 - 292, XP002481763
- TACHIBANA A. ET AL.: 'LidNA, a novel miRNA inhibitor constructed with unmodified DNA' FEBS LETT vol. 586, 2012, pages 1529 - 1532, XP028488271
- Margaret S Ebert ET AL: "MicroRNA sponges: competitive inhibitors of small RNAs in mammalian cells", NATURE METHODS, vol. 4, no. 9, 12 August 2007 (2007-08-12) , pages 721-726, XP055248938, GB ISSN: 1548-7091, DOI: 10.1038/nmeth1079

## Description

### Technical Field

The present invention relates to a nucleic acid molecule that can specifically inhibit the activity of a target RNAi molecule and a pharmaceutical composition containing the nucleic acid molecule as an active ingredient.

### Background Art

Recently, it has been revealed that so-called non-coding RNA not encoding proteins, such as microRNA (miRNA) and small interfering RNA (siRNA), has bioactivities and has various important functions *in vivo.* For example, Non Patent Literature 1 discloses that expression of miR-21, one type of miRNA and called oncomir (cancer miRNA), in a mouse induces pre-B-cell lymphoma. On the other hand, it is also known that many cancer cells express a large amount of miR-21 and that suppression of the expression causes cell death in cancer cell lines such as HeLa cells and human glioma cell U87 (Non Patent Literatures 2 and 3). If a drug that can control the activity of non-coding RNA, such as miRNA, is developed, such a drug can be used as an active ingredient of a medicine for treating various diseases such as cancers or of a diagnostic drug. Accordingly, nucleic acid medicines containing drugs that control the activities of non-coding RNA have been actively being researched and developed in countries all over the world. Various nucleic acid medicines, such as miRNA inhibitors, have been already developed.

For example, Non Patent Literature 4 discloses a method using an artificially constructed non-natural nucleic acid, a bridged nucleic acid (BNA) (locked nucleic acid (LNA)), as a miRNA inhibitor. Non Patent Literature 5 discloses a method using nucleic acid containing RNA chemically modified with 2'-OMe as a miRNA inhibitor. It is generally known that the Tm value between RNA and non-natural nucleic acid or chemically modified nucleic acid is higher than that between RNA and DNA, and the above-mentioned methods inhibit the activity of target miRNA by means of the high binding affinity to RNA. However, the synthesis of non-natural nucleic acid or chemically modified nucleic acid is expensive, i.e., the cost is dozens of times that of unmodified DNA synthesis. Thus, there is a disadvantage that inexpensive mass production is impossible. In addition, application of non-natural nucleic acid or chemically modified nucleic acid, which is not degraded *in vivo,* to medicine involves a serious problem in safety, such as side effects.

Furthermore, miRNA inhibitors consisting of nucleic acid molecules having specific structures are also known. For example, Non Patent Literature 6 discloses an RNA decoy, as a miRNA inhibitor, having a structure including a miRNA-biding site (MBS) between stems. Non Patent Literature 7 discloses a miRNA sponge, as a miRNA inhibitor, having stems on both sides of a complementary sequence having a bulge. However, these miRNA inhibitors are composed of RNA expressed from plasmid vectors and, therefore, are easily degraded by nucleolytic enzymes, such as nucleases, *in vivo* and are very unstable, which is disadvantageous for efficiently and continuously utilizing the pharmacological effects of nucleic acid medicines.

Patent Literature 1 and Non Patent Literatures 8 and 9 disclose miRNA inhibitors each having a stem structure including a target sequence of miRNA and composed of RNA chemically modified with 2'-OMe. In these miRNA inhibitors, the degradation resistance against nucleases is improved by modifying the RNA with 2'-OMe. However, in the point of chemical modification of the RNA, these inhibitors are the same as the above-described chemically modified nucleic acid and therefore still have problems of the cost for synthesis and side effects.

Accordingly, it is demanded to develop a novel nucleic acid medicine that is composed of natural nucleic acid as much as possible, has high degradation resistance against nucleolytic enzymes, can persist relatively stably *in vivo,* and can be provided inexpensively.

Non Patent Literature 10 evaluates the effectiveness of various chemically modified anti-miRNA oligonucleotides (AMOs) for use in cultured cells and rodent models.

Non Patent Literature 11 reviews the chemical modification and design of AMOs.

Non Patent Literature 12 evaluates the *in vivo* potency of different AMOs using various design and modification strategies.

Non Patent Literature 13 discloses the analysis of the cellular function of miRNAs by injecting different AMOs in *Drosophila* embryos.

### Citation List

### Patent Literature

Patent Literature 1: International Publication No. WO2007095387

### Non Patent Literature

Non Patent Literature 1: Medina P.P., et al., 2010, Nature, 467: 86-90
Non Patent Literature 2: Yao Q., et al., 2009, Biochem. Biophys. Res. Commun., 388(3): 539-42
Non Patent Literature 3: Zhou X., et al., 2010, Oncol. Rep., 24(1): 195-201
Non Patent Literature 4: Elmen J., et al., 2008, Nature, 452(7189): 896-9
Non Patent Literature 5: Hutvagner G., et al., 2004, PLoS Biol., 2(4): E98
Non Patent Literature 6: Haraguchi T., et al., 2009, Nucleic Acid Res., 2009, Vol. 37, No. 6, e43
Non Patent Literature 7: Ebert M.S., et al., 2007, Nature Methods, 4: 721-726
Non Patent Literature 8: Vermeulen A., et al., 2007, RNA, 13: 723-730
Non Patent Literature 9: Robertson B., et al., 2010, Silence, 2010, 1: 10
Non Patent Literature 10: Esau C., 2008, Methods, 44: 55-60
Non Patent Literature 11: Lennox K.A. and Mehlke M.A., 2011, Gene Therapy, 18: 1111-1120
Non Patent Literature 12: Lennox K.A. and Mehlke M.A., 2010, Pharm. Res., 27: 1788-1799
Non Patent Literature 13: Boutla A., et al., 2003, Nucleic Acids Research, 31(17): 4973-4980

### Summary of Invention

### Technical Problem

In development of a nucleic acid medicine, from the viewpoint of cost and safety to subjects, it is better that the nucleic acid medicine be composed of natural nucleic acid capable of being degraded *in vivo,* i.e., RNA or DNA, than that the medicine is composed of non-natural nucleic acid or chemically modified nucleic acid. Here, RNA has high binding affinity to non-coding RNA such as miRNA and therefore has high inhibitory activity. However, RNA has disadvantages: high instability *in vivo,* low chemical synthesis efficiency, and relatively high synthesis cost, though it is not as high as in non-natural nucleic acid or chemically modified nucleic acid. On the other hand, DNA is relatively stable *in vivo* compared to RNA and can be synthesized with the lowest cost compared to other nucleic acid. However, the binding affinity to non-coding RNA is low. In particular, DNA in a low concentration cannot bind to non-coding RNA such as miRNA even if the nucleotide sequences are complementary to each other. Therefore, DNA has a disadvantage of low inhibitory activity.

It is an object of the present invention to develop and provide a nucleic acid molecule that can specifically and efficiently inhibit the activity of a target RNAi molecule and can be produced in large quantities safely and inexpensively.

### Solution to Problem

The present inventors have intensively studied to solve the above problems and have found that even if the nucleic acid having a nucleotide sequence complementary to the nucleotide sequence of a functional strand in an RNAi molecule is composed of DNA, the binding affinity to the nucleotide sequence of the functional strand can be dramatically increased by linking a double-stranded nucleic acid to at least one of the 5'-end and 3'-end of the DNA and that, as a result, the gene silencing activity of the RNAi molecule can be inhibited. The present invention was accomplished based on these and other findings.

The invention provides a nucleic acid molecule for inhibiting an activity of a target RNAi molecule, and a pharmaceutical composition comprising the nucleic acid molecule, both as defined in the claims.

### Advantageous Effects of Invention

The nucleic acid molecule of the present invention can specifically and efficiently inhibit the activity of a target RNAi molecule. In addition, the nucleic acid molecule of the present invention, even if it is composed of DNA only, can bind to a target RNAi molecule to inhibit the activity of the target RNAi molecule. Accordingly, the nucleic acid molecule can be highly safe and can be chemically synthesized simply, in a large quantity, and inexpensively.

### Brief Description of Drawings

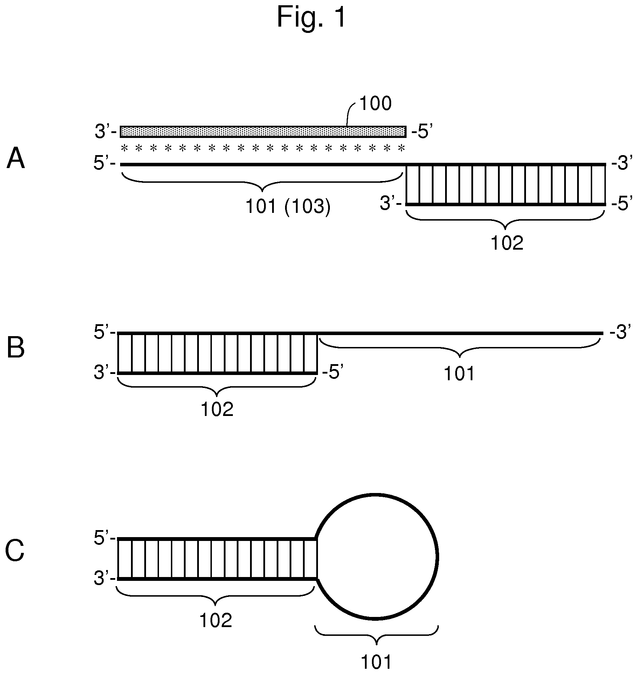

[Figure 1] Figure 1 includes conceptual diagrams illustrating the structures of the nucleic acid molecule for inhibiting the activity of an RNAi molecule of the present invention. These diagrams each illustrate minimum and indispensable structural units of the nucleic acid molecule for inhibiting the activity of an RNAi molecule of the present invention. Diagram A illustrates a structure in which a double-stranded nucleic acid moiety (102) is linked to the 3'-end of a single-stranded nucleic acid moiety (101); diagram B illustrates a structure in which a double-stranded nucleic acid moiety (102) is linked to the 5'-end of a single-stranded nucleic acid moiety (101); and diagram C illustrates a structure in which the 5'-end and the 3'-end of a single-stranded nucleic acid moiety (101) are linked to the 3'-end and the 5'-end of a double-stranded nucleic acid moiety (102), respectively. In diagram A, the single-stranded nucleic acid moiety (101) is composed of only a nucleotide sequence complementary to a target RNAi molecule (100), and the whole region of the single-stranded nucleic acid moiety (101) corresponds to an unmodified DNA region (103). In diagrams A to C, the vertical bars denote base pairs between nucleic acid strands of a double-stranded nucleic acid moiety (102); and the stars denote base pairs between a single-stranded nucleic acid moiety (101) and a target RNAi molecule (100) (the same shall apply hereinafter).

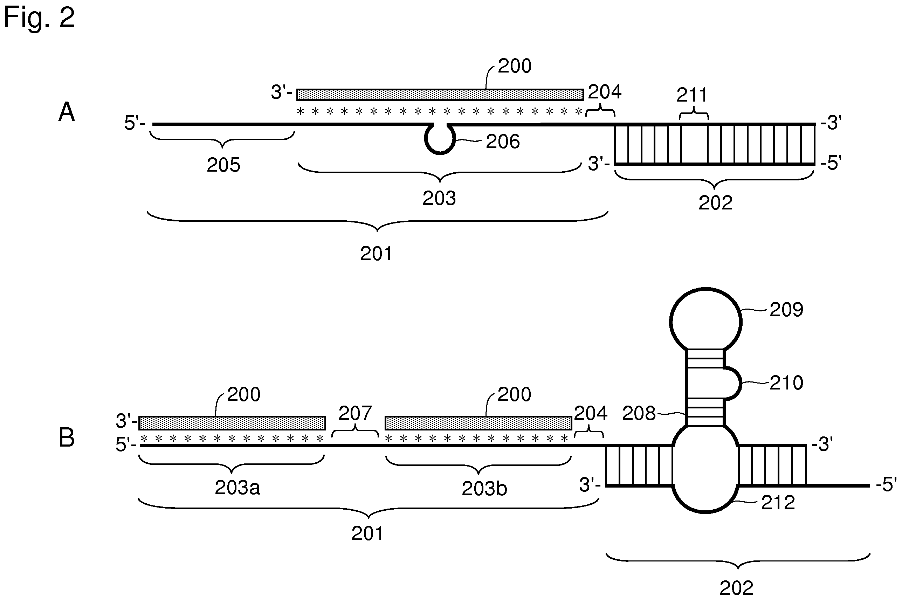

[Figure 2] Figure 2 includes conceptual diagrams illustrating optional structural elements in the structural unit of the nucleic acid molecule for inhibiting the activity of an RNAi molecule of the present invention, such as a linking region (204) linking a single-stranded nucleic acid moiety (201) and a double-stranded nucleic acid moiety (202), a flanking region (205), a mismatch site (206), and a spacer region (207) between two or more unmodified DNA regions (203a and 203b).

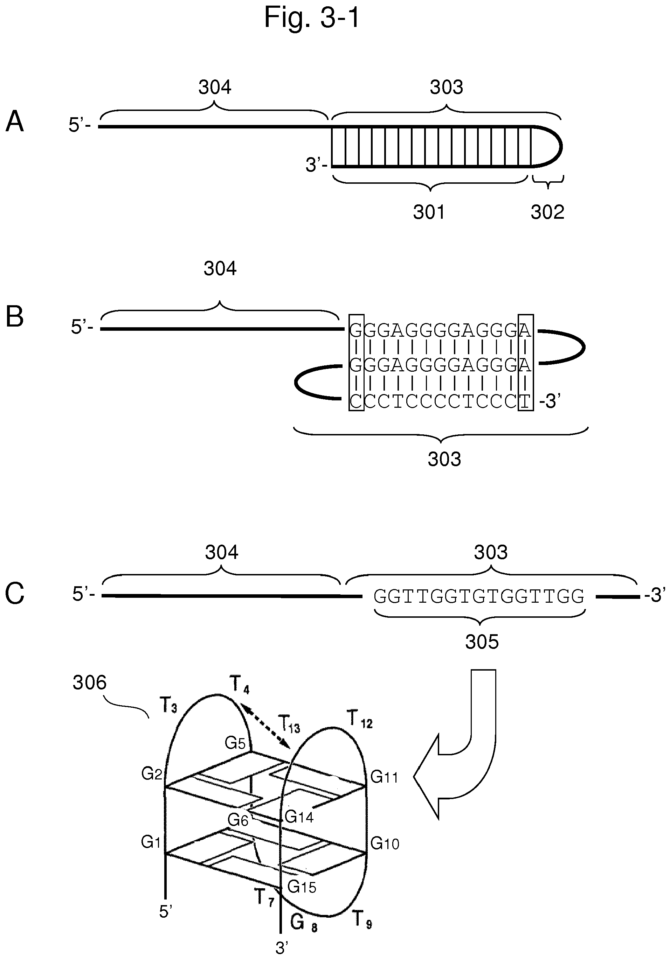

[Figure 3-1] Figure 3-1 shows examples of specific structures in which the double-stranded nucleic acid moiety of the nucleic acid molecule for inhibiting the activity of an RNAi molecule of the present invention is composed of a single nucleic acid strand. Diagram A shows an example of a single nucleic acid strand constructs a single-stranded nucleic acid moiety (304) and a double-stranded nucleic acid moiety (303) by linking two complementary nucleic acid strand regions (301) with a loop region (302). Diagram B shows an example where a single nucleic acid strand constituting a double-stranded nucleic acid moiety includes a specific sequence in the nucleotide sequence to form a triplex. In the example of three rows of nucleic acid strands shown in the diagram, each three nucleotides (GGC or AAT) surrounded by a frame forms base pairs with each other, and thereby three nucleic acid strands form a triple strand. Diagram C shows an example where a single nucleic acid strand constituting a double-stranded nucleic acid moiety includes a specific sequence in the nucleotide sequence to form a quadruplex. Here, in the example shown in the diagram, the guanine-rich sequence (305) of the double-stranded nucleic acid moiety forms a G-quartet (306).

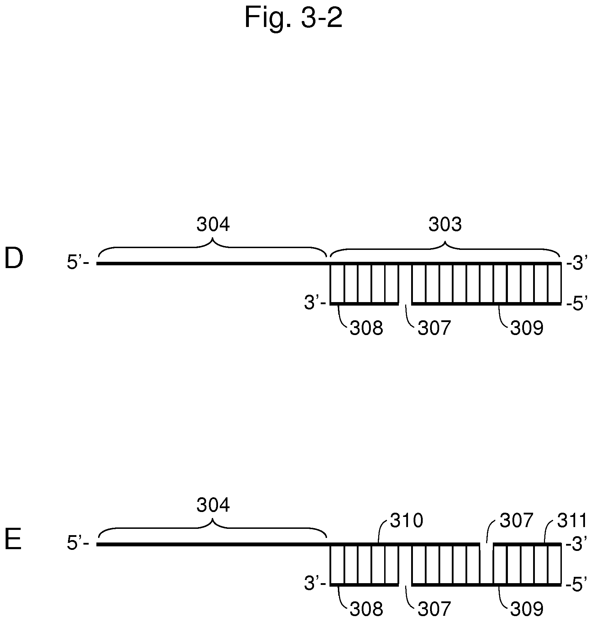

[Figure 3-2] Figure 3-2 shows examples of specific structures in which the double-stranded nucleic acid moiety of the nucleic acid molecule for inhibiting the activity of an RNAi molecule of the present invention is composed of a single nucleic acid strand. Diagram D shows a constitutional example in which the double-stranded nucleic acid moiety (303) has a nick (307) in one of the two nucleic acid strands complementary to each other forming base pairs. Diagram E shows a constitutional example in which the double-stranded nucleic acid moiety (303) has nicks (307) in both of the two nucleic acid strands complementary to each other forming base pairs.

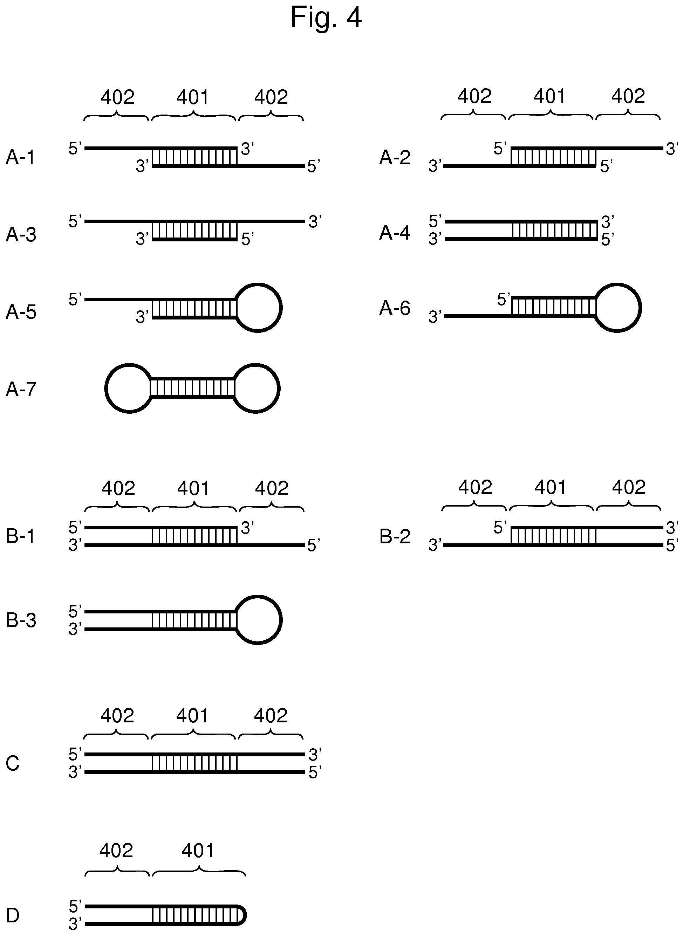

[Figure 4] Figure 4 includes conceptual diagrams illustrating structural embodiments of the nucleic acid molecule of the present invention, comprising one double-stranded nucleic acid moiety (401) and two (A-1 to A-7 and D), three (B-1 to B-3), or four (C) single-stranded nucleic acid moieties (402) in one molecule. Diagrams A-1 to A-7, B-1 to B-3, and C show structural embodiments where the double-stranded nucleic acid moiety is composed of two nucleic acid strands. Diagram D shows a structural embodiment where the double-stranded nucleic acid moiety is composed of a single nucleic acid strand.

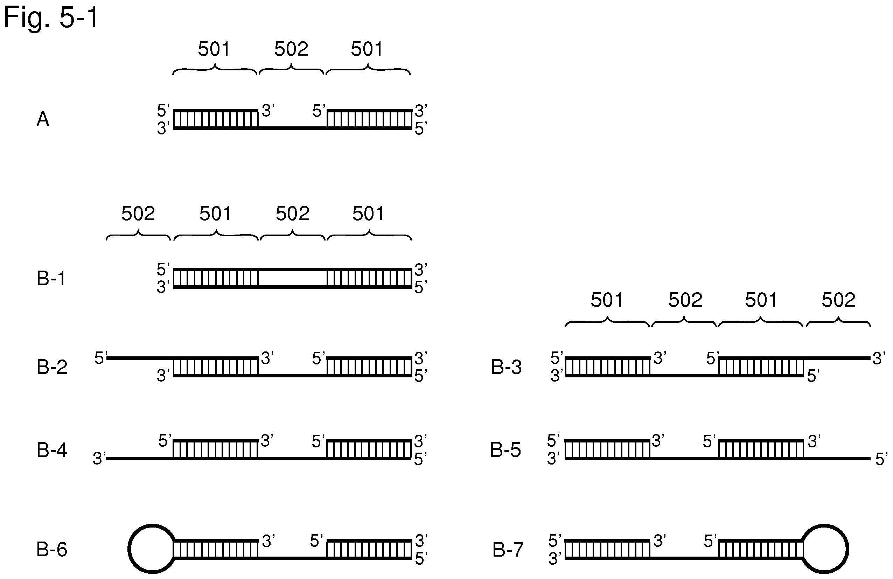

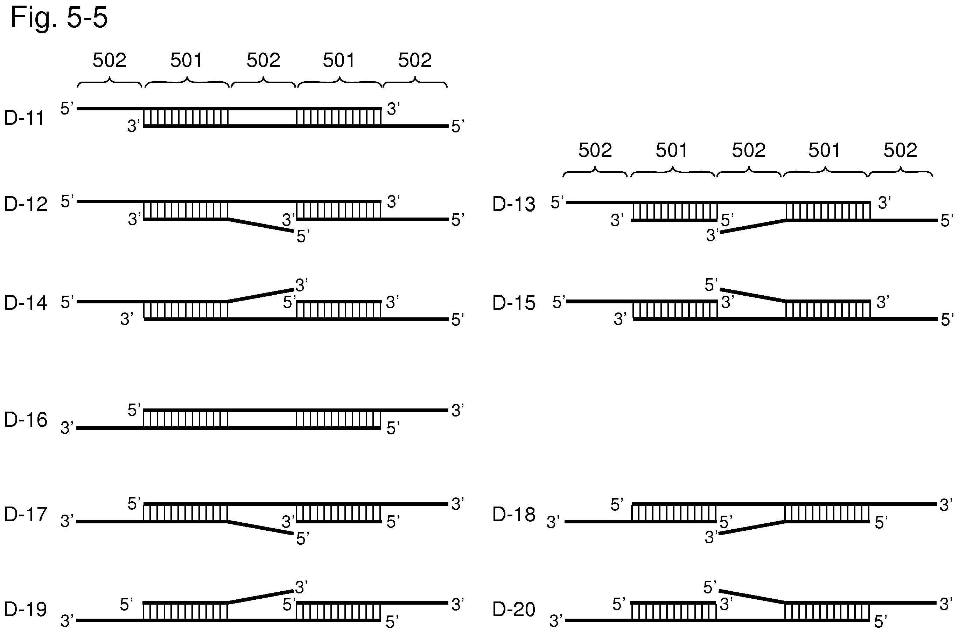

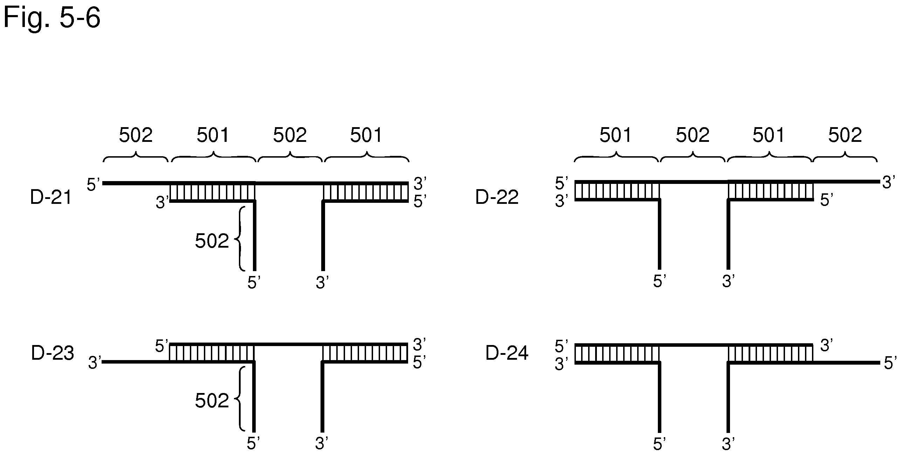

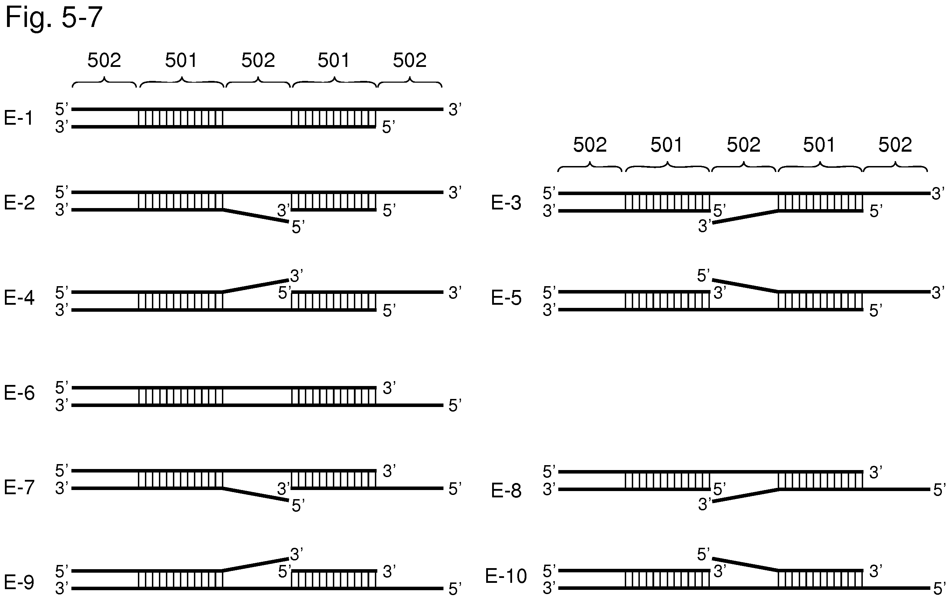

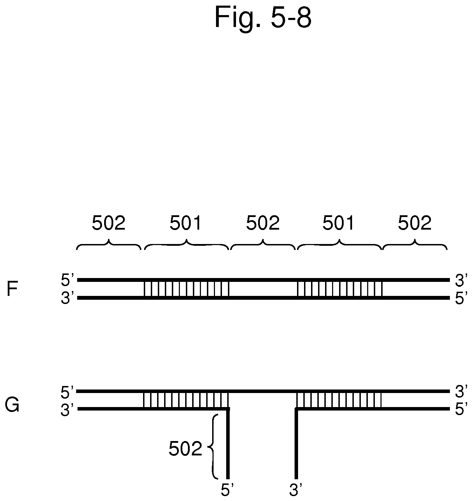

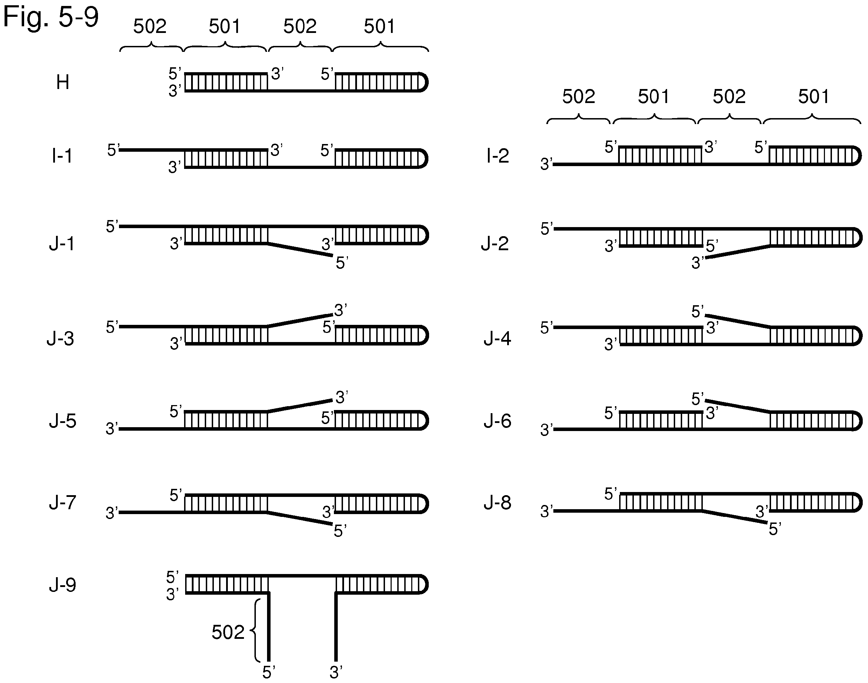

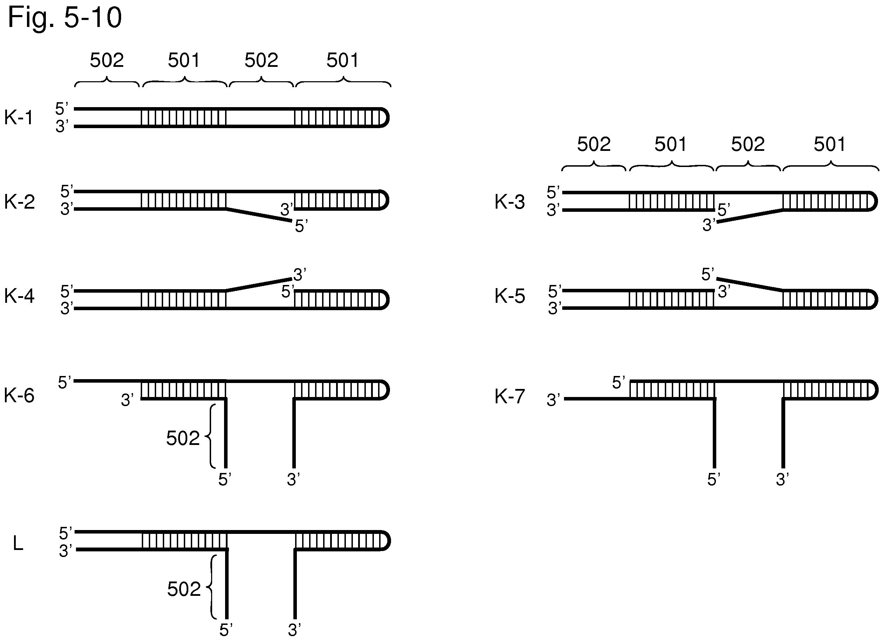

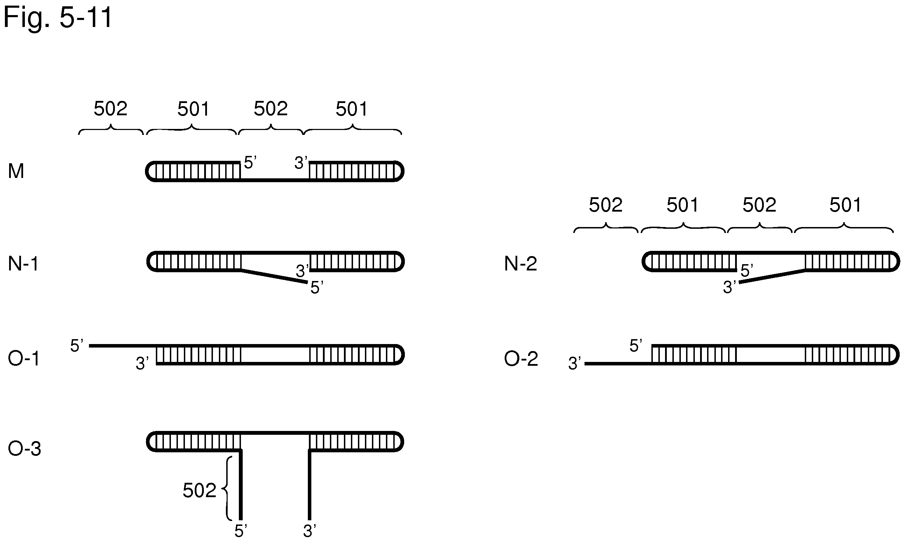

[Figure 5-1] Figure 5-1 includes conceptual diagrams illustrating structural embodiments of the nucleic acid molecule of the present invention, comprising two double-stranded nucleic acid moieties (501) and one (A) or two (B-1 to B-7) single-stranded nucleic acid moieties (502) in one molecule. These diagrams show structural embodiments in which two double-stranded nucleic acid moieties are each composed of two nucleic acid strands.

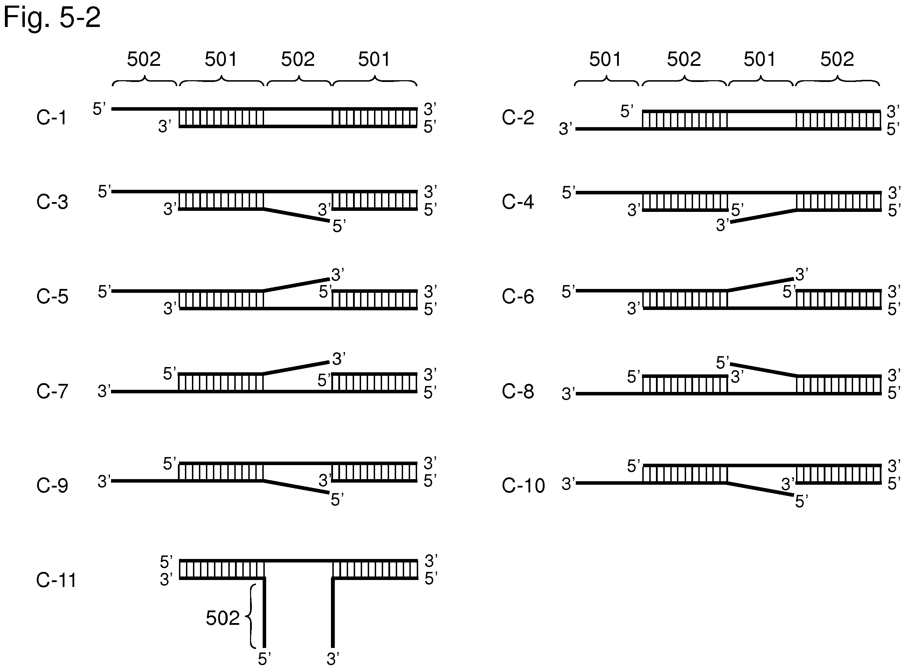

[Figure 5-2] Figure 5-2 includes conceptual diagrams partially illustrating structural embodiments of the nucleic acid molecule of the present invention, comprising two double-stranded nucleic acid moieties (501) and three (C-1 to C-11) single-stranded nucleic acid moieties (502) in one molecule. These diagrams show structural embodiments in which two double-stranded nucleic acid moieties are each composed of two nucleic acid strands.

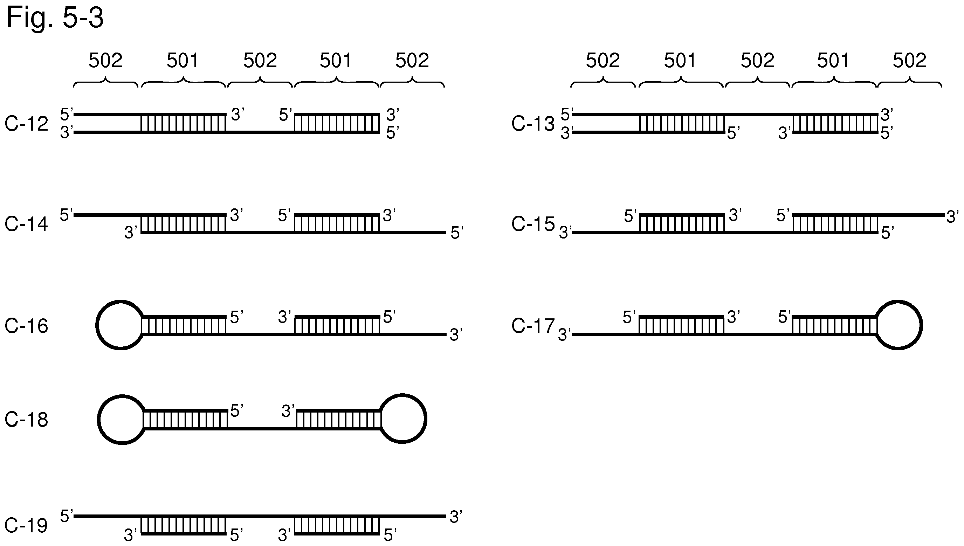

[Figure 5-3] Figure 5-3 includes conceptual diagrams partially illustrating structural embodiments of the nucleic acid molecule of the present invention, comprising two double-stranded nucleic acid moieties (501) and three (C-12 to C-19) single-stranded nucleic acid moieties (502) in one molecule. These diagrams show structural embodiments in which two double-stranded nucleic acid moieties are each composed of two nucleic acid strands.

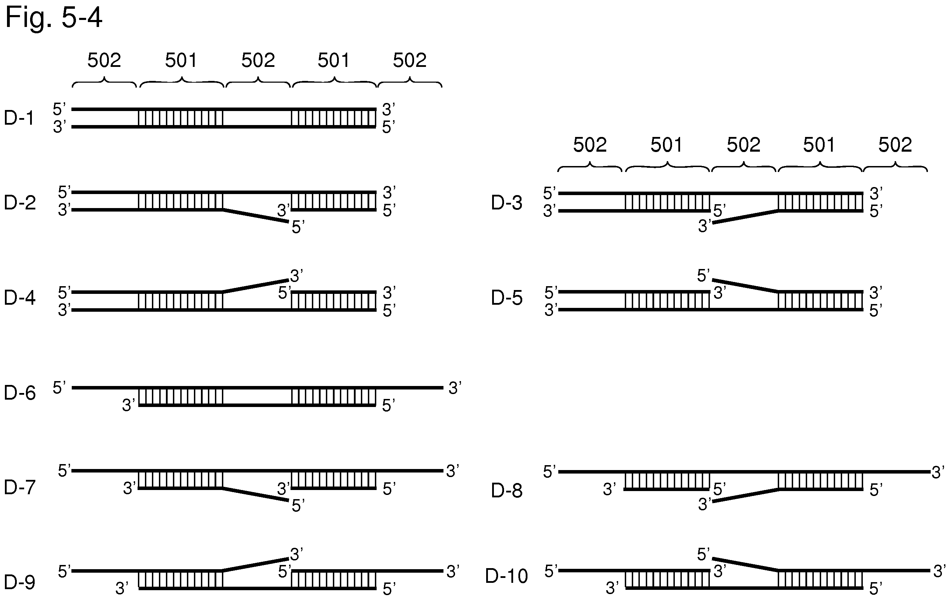

[Figure 5-4] Figure 5-4 includes conceptual diagrams partially illustrating structural embodiments of the nucleic acid molecule of the present invention, comprising two double-stranded nucleic acid moieties (501) and four (D-1 to D-10) single-stranded nucleic acid moieties (502) in one molecule. These diagrams show structural embodiments in which two double-stranded nucleic acid moieties are each composed of two nucleic acid strands.
[Figure 5-5] Figure 5-5 includes conceptual diagrams partially illustrating structural embodiments of the nucleic acid molecule of the present invention, comprising two double-stranded nucleic acid moieties (501) and four (D-11 to D-20) single-stranded nucleic acid moieties (502) in one molecule. These diagrams show structural embodiments in which two double-stranded nucleic acid moieties are each composed of two nucleic acid strands.
[Figure 5-6] Figure 5-6 includes conceptual diagrams partially illustrating structural embodiments of the nucleic acid molecule of the present invention, comprising two double-stranded nucleic acid moieties (501) and four (D-21 to D-24) single-stranded nucleic acid moieties (502) in one molecule. These diagrams show structural embodiments in which two double-stranded nucleic acid moieties are each composed of two nucleic acid strands.
[Figure 5-7] Figure 5-7 includes conceptual diagrams partially illustrating structural embodiments of the nucleic acid molecule of the present invention, comprising two double-stranded nucleic acid moieties (501) and five (E-1 to E-10) single-stranded nucleic acid moieties (502) in one molecule. These diagrams show structural embodiments in which two double-stranded nucleic acid moieties are each composed of two nucleic acid strands.
[Figure 5-8] Figure 5-8 includes conceptual diagrams partially illustrating structural embodiments of the nucleic acid molecule of the present invention, comprising two double-stranded nucleic acid moieties (501) and six (F) or seven (G) single-stranded nucleic acid moieties (502) in one molecule. These diagrams show structural embodiments in which two double-stranded nucleic acid moieties are each composed of two nucleic acid strands.
[Figure 5-9] Figure 5-9 includes conceptual diagrams illustrating structural embodiments of the nucleic acid molecule of the present invention, comprising two double-stranded nucleic acid moieties (501) and one (H), two (1-1 and 1-2), or three (J-1 to J-9) single-stranded nucleic acid moieties (502) in one molecule. These diagrams show structural embodiments in which one of two double-stranded nucleic acid moieties is composed of a single nucleic acid strand.
[Figure 5-10] Figure 5-10 includes conceptual diagrams illustrating structural embodiments of the nucleic acid molecule of the present invention, comprising two double-stranded nucleic acid moieties (501) and four (K-1 to K-7) or five (L) single-stranded nucleic acid moieties (502) in one molecule. These diagrams show structural embodiments in which one of two double-stranded nucleic acid moieties is composed of a single nucleic acid strand.
[Figure 5-11] Figure 5-11 includes conceptual diagrams illustrating structural embodiments of the nucleic acid molecule of the present invention, comprising two double-stranded nucleic acid moieties (501) and one (M), two (N-1 and N-2), or three (O-1 to O-3) single-stranded nucleic acid moieties (502) in one molecule. These diagrams show structural embodiments in which both of two double-stranded nucleic acid moieties are each composed of a single nucleic acid strand.
[Figure 6] Figure 6 is a conceptual diagram illustrating a structural embodiment of the nucleic acid molecule comprising three or more double-stranded nucleic acid moieties and three or more single-stranded nucleic acid moieties in one molecule.
[Figure 7-1] Figure 7-1 shows names, structures, nucleotide sequences, and SEQ ID NOs of nucleic acid molecules used in Example 1 and other examples. In the diagram, DNA is given in capital letters, and RNA is given in small letters. The bold letters denote single-stranded nucleic acid moieties, where mismatch sites are underlined, and linking regions are double-underlined. Accordingly, the bold letters not underlined denote unmodified DNA regions. The thin letters denote double-stranded nucleic acid moieties. The same shall apply to Figures 7-2 to 7-7.
[Figure 7-2] Figure 7-2 shows names, structures, nucleotide sequences, and SEQ ID NOs of nucleic acid molecules used in Example 1 and other examples.
[Figure 7-3] Figure 7-3 shows names, structures, nucleotide sequences, and SEQ ID NOs of nucleic acid molecules used in Example 1 and other examples.
[Figure 7-4] Figure 7-4 shows names, structures, nucleotide sequences, and SEQ ID NOs of nucleic acid molecules used in Example 1 and other examples.
[Figure 7-5] Figure 7-5 shows names, structures, nucleotide sequences, and SEQ ID NOs of nucleic acid molecules used in Example 4.
[Figure 7-6] Figure 7-6 shows names, structures, nucleotide sequences, and SEQ ID NOs of nucleic acid molecules used in Examples 5 and 7 and other examples.
[Figure 7-7] Figure 7-7 shows names, structures, nucleotide sequences, and SEQ ID NOs of nucleic acid molecules used in Example 6.

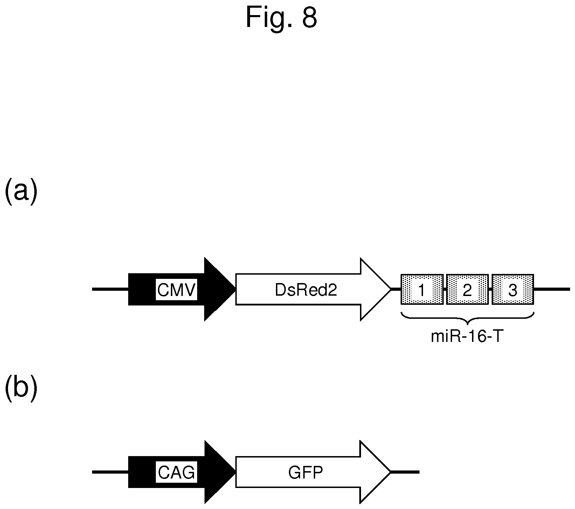

[Figure 8] Figure 8 shows the structure of the measuring system used in Example 1. Diagram (a) is a conceptual diagram partially illustrating a main structure of pDsRed2-mi16-T of which the effect of inhibiting the activity of miR-16 is measured. In this expression vector, three-times repeated miR-16-target (miR-16-T) is inserted, which is a target site of miR-16 having a sequence completely complementary to miR-16. Diagram (b) is a conceptual diagram partially illustrating a main structure of pCAGGS-AFP, which is a GFP expression vector for correcting the effect of inhibiting the activity of miR-16.
[Figure 9] Figure 9 is a graph showing the effect of inhibiting the activity of miR-16 by each nucleic acid molecule in Example 1. In the graph, the names of nucleic acid molecules shown in the horizontal axis correspond to the names of the nucleic acid molecules in Figures 7-1 to 7-4. The vertical axis shows relative values when the normalized DsRed2/GFP ratio in the above-described control is defined as 1.
[Figure 10] Figure 10 shows the effect of inhibiting the activity of miR-16 by each nucleic acid molecule in Example 2.
[Figure 11] Figure 11 shows the effect of inhibiting the activity of miR-16 -by the nucleic acid molecule for inhibiting the activity of an RNAi molecule, sp-mi16-11, in each cultured cell in Example 3.
[Figure 12] Figure 12 shows a relationship between the nucleotide length of a mismatch site and the effect of inhibiting the activity in the nucleic acid molecules of the present invention. The number in parentheses of the name of each nucleic acid molecule means the number of nucleotides of the mismatch site.
[Figure 13] Figure 13 shows a relationship between the position of a mismatch site and the effect of inhibiting the activity in the nucleic acid molecules of the present invention. The number in parentheses of the name of each nucleic acid molecule means the number of nucleotides of the mismatch site. The structure of sp-miR16-17(12) corresponds to nucleic acid molecule sp-miR16-11 shown in Figure 7-3.
[Figure 14] Figure 14 shows a relationship between the nucleotide length of a linking region and the effect of inhibiting the activity in the nucleic acid molecules of the present invention. The number in parentheses of the name of each nucleic acid molecule means the number of nucleotides of the linking region.
[Figure 15] Figure 15 is graph showing the effect of inhibiting the activity of miR-143 by each nucleic acid molecule in Example 7. In the graph, the names of nucleic acid molecules, sp-miR143-1 and sp-miR21-1, shown in the horizontal axis correspond to the names of the nucleic acid molecules in Figure 7-6. The vertical axis shows relative values when the normalized DsRed2/GFP ratio in the control not being added the nucleic acid molecule of the present invention is defined as 1.

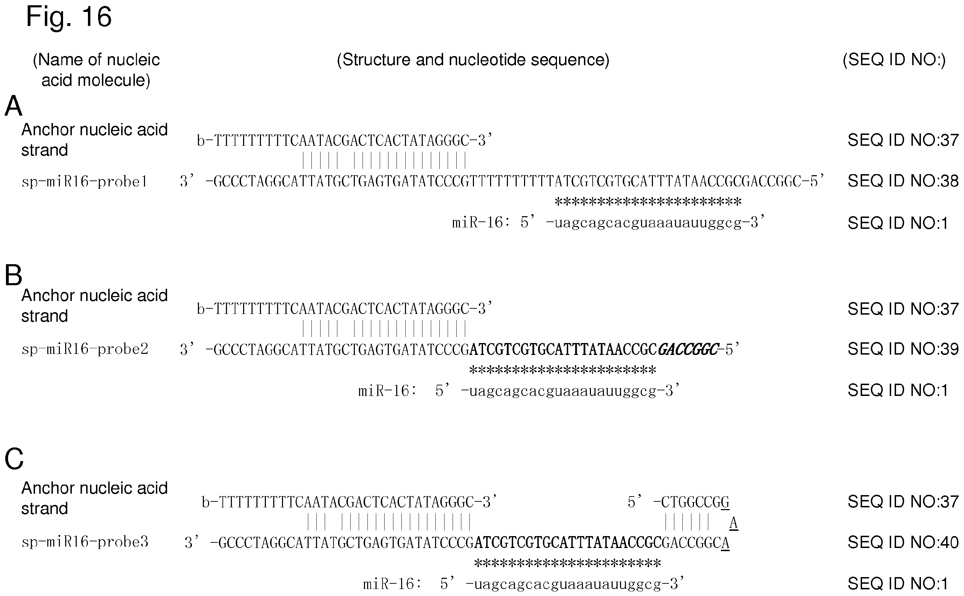

[Figure 16] Figure 16 shows nucleic acid molecules used in Example 8. In the diagram, DNA is given in capital letters, and RNA is given in small letters. In diagrams B and C, the bold letters denote single-stranded nucleic acid moieties, and the thin letters denote double-stranded nucleic acid moieties. In diagram B, the italic letters denote a flanking region, and the underlined letters in diagram B denote a loop region. That is, diagram A shows the structure and the nucleotide sequence of a nucleic acid molecule, sp-miR16-probel, formed of only a single-stranded nucleic acid moiety, not having any double-stranded nucleic acid moiety; diagram B shows the structure and the nucleotide sequence of sp-miR16-probe2 composed of a single-stranded nucleic acid moiety and a double-stranded nucleic acid moiety consisting of two nucleic acid strands at the 3'-end; and diagram C shows the structure and the nucleotide sequence of sp-miR16-probe3 consisting of a single-stranded nucleic acid moiety, a double-stranded nucleic acid moiety consists of two nucleic acid strands at the 5'-end, and a single nucleic acid strand having a loop region at the 3'-end, respectively. The "b" shown at the 5'-end part of each anchor nucleic acid strand means biotinylation.
[Figure 17] Figure 17 shows conceptual diagrams of each nucleic acid molecule shown in Figure 16 and the avidities of sp-miR16-probe1 to sp-miR16-probe3 to miRNA measured by an SPR method. Diagrams A to C respectively show the results of sp-miR16-probel, sp-miR16-probe2, and sp-miR16-probe3, wherein the concentrations (a) to (e) are 50 nM, 25 nM, 12.5 nM, 6.25 nM, and 3.125 nM.

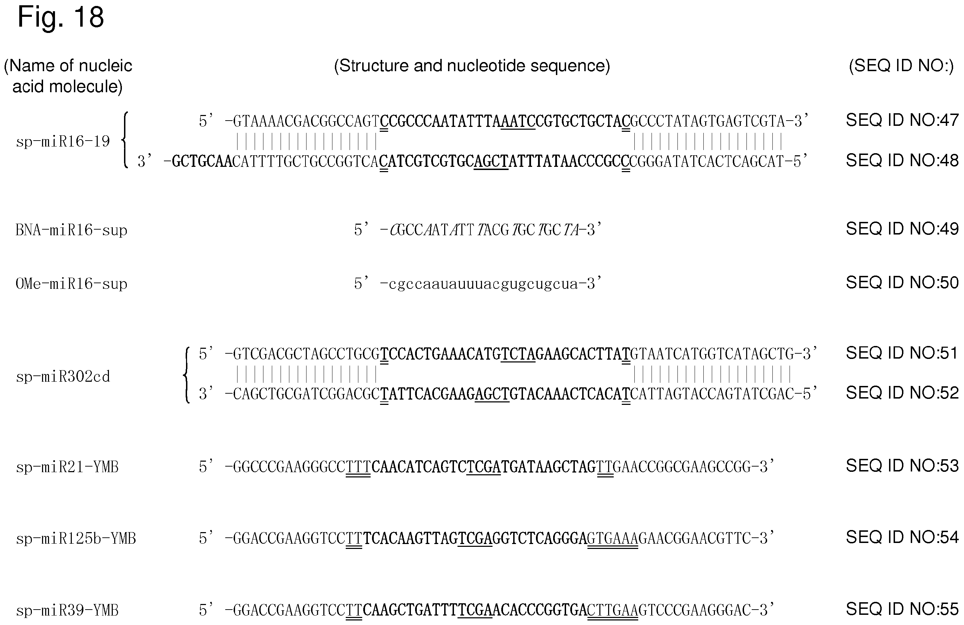

[Figure 18] Figure 18 shows names, structures, nucleotide sequences, and SEQ ID NOs of nucleic acid molecules used in Example 9. In the diagram, in sp-miR16-19 and sp-miR302cd, the capital letters denote DNA, and the bold letters denote single-stranded nucleic acid moieties, where mismatch sites are underlined, and linking regions are double-underlined. Accordingly, the bold letters not underlined denote unmodified DNA regions. The thin letters denote double-stranded nucleic acid moieties. In BNA-miR16-sup, the italic letters denote a site composed of non-natural nucleic acid, BNA; and other sites are composed of DNA. OMe-miR16-sup wholly consists of RNA chemically modified by 2'-OMe, wherein the RNA is given in small letters. The vertical axis shows relative values when the normalized DsRed2/GFP ratio in sp-miR302cd as a control is defined as 1.
[Figure 19] Figure 19 shows the effect of inhibiting the activity of endogenous miR-16 by each nucleic acid molecule in Example 9.
[Figure 20] Figure 20 is a graph showing that the nucleic acid molecule for inhibiting the activity of miR-21, sp-miR21-1, inhibits the activity of miR-21, which is known as an oncomir (cancer miRNA) highly expressed in cancer cells and is present in breast cancer cell line MCF7, and thereby suppresses proliferation of MCF7 cells.
[Figure 21] Figure 21 is a graph showing suppression of proliferation of MCF7 cells by the nucleic acid molecule for inhibiting the activity of miR-21, sp-miR21-YMB, which has a structure different from that of sp-miR21-1.
[Figure 22] Figure 22 is a graph showing the effect of inhibiting the activity of exogenous miR-125b by each nucleic acid molecule in Example 12.

### Description of Embodiments

### 1. Nucleic acid molecule for inhibiting the activity of an RNAi molecule

### 1-1. Summary

A first embodiment of the present invention relates to a nucleic acid molecule for inhibiting the activity of a target RNAi molecule. The nucleic acid molecule for inhibiting the activity of an RNAi molecule of the present invention (hereinafter, throughout the present specification, often shortened to "nucleic acid molecule") is inexpensive and can be easily synthesized and also can specifically inhibit the activity of a target RNAi molecule.

### 1-2. Definition

In the present invention, the term "RNAi molecule" refers to an RNA molecule that can induce RNA interference (RNAi) *in vivo* and inhibit the expression of a target gene (silencing) through degradation of a transcriptional product of the gene (Fire A. et al., 1998, Nature, 391, 806-811). In the present invention, the term "target RNAi molecule" is a target of the nucleic acid molecule of the present invention and refers to an RNAi molecule as the target of which the activity should be inhibited. Specific examples of the RNAi molecule include siRNA, miRNA, and shRNA.

The term "siRN (small interference RNA)" refers to small double-stranded RNA consisting of a sense strand (passenger strand) including a nucleotide sequence corresponding to a part of a target gene and an antisense strand (guide strand) thereof.

The term "miRNA (micro RNA)" refers to single-stranded non-coding RNA present *in vivo,* having a length of 18 to 25 nucleotides, and controlling the expression of a specific gene. The RNA is known to bind to the mRNA and the protein factor of a target gene to form a complex and thereby inhibit the target gene from being translated. iRNA is transcribed from a genome in a single-stranded precursor state, called pri-miRNA, and is further processed by an endonuclease, called Drosha, into a single-stranded precursor state, called pre-miRNA, inside the nucleus, and is formed into a mature double-stranded miRNA consisting of a miRNA strand and a miRNA star strand by the action of an endonuclease, called Dicer, outside the nucleus. The miRNA strand is incorporated into an RNA-induced silencing complex (RISC) to form mature single-stranded miRNA to inhibit the target gene expression (David P. Bartel, Cell, Vol. 116, 281-297, January 23, 2004,).

The term "shRNA (short hairpin RNA)" refers to single-stranded RNA in which a sense strand and an antisense strand, of the siRNA or the mature double-stranded miRNA, are linked with a short spacer sequence having an appropriate sequence. That is, in shRNA, the sense region and the antisense region are base-paired with each other to form a stem structure, and simultaneously, the spacer sequence forms a loop structure. Consequently, in one molecule, a hairpin-shaped stem-and-loop structure is formed as a whole molecule.

In the present invention, the term "the activity of an RNAi molecule" refers to the activity of an RNAi molecule for silencing the expression of a target gene (gene silencing activity). In the present invention, the term "inhibiting the activity of an RNAi molecule" refers to completely or partially inhibiting the gene silencing activity of an RNAi molecule. The RNAi molecule may be an endogenous RNAi molecule or an exogenous RNAi molecule. When the activity of a specific RNAi molecule present in the living body (including in a cell, tissue, organ, or individual) is inhibited, the silencing of a gene as a target of the specific RNAi molecule is relatively completely or partially avoided. As a result, the expression level of the target gene *in vivo* is increased.

In the present invention, the term "nucleic acid" refers to a biological macromolecule, of which the structural unit is nucleotides linked to one another by phosphodiester bonds. In principle, nucleic acid refers to a natural nucleic acid consisting of natural nucleotides present in nature. The nucleic acid molecule of the present invention encompasses natural nucleic acid, artificial nucleic acid, and mixtures thereof.

Throughout the present specification, the term "natural nucleotide" is a deoxyribonucleotide having a base of any of adenine, guanine, cytosine, and thymine or a ribonucleotide having a base of any of adenine, guanine, cytosine, and uracil. The term "natural nucleic acid" is DNA composed of linked deoxyribonucleotides or RNA composed of linked ribonucleotides.

Throughout the present specification, the term "artificial nucleic acid" refers a nucleic acid wholly or partially composed of non-natural nucleotides or a non-natural nucleic acid.

Throughout the present specification, the term "non-natural nucleotide" refers to an artificially constructed nucleotide that is not present in nature. That is, the non-natural nucleotide is an artificial nucleotide having a property and/or structure similar to that of a natural nucleotide or an artificial nucleotide including a non-natural nucleoside or a non-natural base, having a property and/or structure similar to that of a natural nucleoside or a natural base as the structural element of a natural nucleotide. Specific examples of the non-natural nucleoside include abasic nucleosides, arabinonucleosides, 2'-deoxyuridine, α-deoxyribonucleoside, and β-L-deoxyribonucleoside. Specific examples of the non-natural base include a 2-oxo(1H)-pyridin-3-yl group, a 5-position substituted 2-oxo(1H)-pyridin-3-yl group, a 2-amino-6-(2-thiazolyl)purin-9-yl group, and a 2-amino-6-(2-oxazolyl)purin-9-yl group.

Throughout the present specification, the term "non-natural nucleic acid" refers to artificially constructed nucleic acid having a structure and/or a property similar to that of natural nucleic acid. Examples of the non-natural nucleic acid include peptide nucleic acid (PNA), peptide nucleic acid having a phosphate group (PHONA), bridged nucleic acid/locked nucleic acid (BNA/LNA), and morpholino nucleic acid.

In addition, the nucleic acid molecule of the present invention may be modified excluding the unmodified DNA region described below. Herein, the term "modification" refers to a part or whole of the nucleotides as a structural unit of nucleic acid or the nucleosides as their structural elements are replaced by other atomic groups or addition of a functional group or the like. Specifically, examples of the modification include glycosylation, base modification, and phosphate modification.

The glycosylation is modification of a ribose moiety constituting a nucleoside. For example, the modification of the ribose moiety constituting a ribonucleoside is substitution of or addition to the hydroxy group at the 2'-position. Specifically, the glycosylated ribose is, for example, 2'-O-methylribose having a methoxy group substituted for the hydroxy group, 2'-O-ethylribose having an ethoxy group substituted for the hydroxy group, 2'-O-propylribose having a propoxy group substituted for the hydroxy group, 2'-O-butylribose having a butoxy group substituted for the hydroxy group, 2'-deoxy-2'-fluororibose having a fluoro group substituted for the hydroxy group, or a 2'-O-methoxyethylribose having a 2'-O-methoxyethyl group substituted for the hydroxy group. Examples of the modification also include substitution of the (deoxy)ribose moiety of a nucleoside with another sugar. Specifically, the modification is, for example, cross-linking of the arabinose of the ribose moiety, 2'-fluoro-β-D-arabinose, or the 2'-hydroxy group of ribose to the carbon atom at the 4'-position via methylene to form a ribose derivative; substitution of the oxygen at the 4-position of a ribose ring with sulfur to form a ribose derivative; or substitution of the oxygen atom on the ribofuranose ring (oxygen atom at the 4-position of ribose) with sulfur.

The base modification is modification of the base moiety constituting a nucleoside. Examples of the modification include substitution of the base moiety for a functional group, addition of a functional group to the base moiety, and substitution of the base moiety with a base analog. Specifically, the modification is, for example, a modified pyrimidine such as 5-methylcytosine having cytosine substituted with a methyl group at the 5-position, 5-hydroxycytosine having cytosine substituted with a hydroxy group at the 5-position, 5-fluorouracil having uracil substituted with a fluoro group at the 5-position, 4-thiouracil having uracil in which the oxygen atom at the 4-position is replaced by a thio group, 5-methyluracil having uracil substituted with a methyl group at the 5-position, or 2-thiouracil having uracil in which the oxygen atom at the 2-position is replaced by a thio group; a modified purine such as 6-methyladenine having adenine substituted with a methyl group at the 6-position, or 6-thioguanine having guanine substituted with a thio group at the 6-position; or another heterocyclic base.

Further, the phosphate group, sugar, and/or base of the nucleic acid molecule of the present invention may be optionally labeled with a nucleic acid-labeling material. The labeling may be applied to the nucleotide constituting an unmodified DNA region described below. The nucleic acid-labeling material may be any material that is known in the art, such as a radioisotope (e.g., ³²P, ³H, or ¹⁴C), DIG, biotin, a fluorescent dye (e.g., FITC, Texas, Cy3, Cy5, Cy7, FAM, HEX, VIC, JOE, Rox, TET, Bodipy493, NBD, or TAMRA), or a luminescent material (e.g., an acridinium ester).

### 1-3. Structure

### 1-3-1. Structural unit

Figures 1 and 2 show the structures of the nucleic acid molecule for inhibiting the activity of an RNAi molecule of the present invention. As shown in these drawings, the nucleic acid molecule of the present invention encompasses a structure comprising, as minimum and indispensable structural units, one single-stranded nucleic acid moiety (101, 201) and one double-stranded nucleic acid moiety (102, 202) linked to one end of the single-stranded nucleic acid moiety. The double-stranded nucleic acid moiety may be linked to the 3'-end (Figure 1A) or the 5'-end (Figure 1B) of the single-stranded nucleic acid moiety, but the position is preferably the 3'-end having higher effect of inhibiting the activity of an RNAi molecule. Alternatively, both ends of the single-stranded nucleic acid moiety may be linked to the double-stranded nucleic acid moiety such that the double-stranded nucleic acid moiety and the single-stranded nucleic acid moiety form a stem structure and a loop structure (Figure 1C).

The single-stranded nucleic acid moiety and the double-stranded nucleic acid moiety will now be specifically described.

### (1) Single-stranded nucleic acid moiety

The "single-stranded nucleic acid moiety" (101, 201) is a nucleic acid moiety consisting of a single strand of the nucleic acid molecule of the present invention and includes at least one, preferably three or less, and more preferably two or less of unmodified DNA regions (103, 203) inside the moiety. In the case of the single-stranded nucleic acid moiety having two or more unmodified DNA regions, the respective unmodified DNA regions (e.g., 203a and 203b) may be the same or different. Herein, "the same" refers to that the target RNAi molecules are the same and/or that the nucleotide sequences constituting the regions are the same. Herein, "different" refers to that the target RNAi molecules are different from each other and/or that the nucleotide sequences constituting the regions are different from each other.

In Figure 1A, the single-stranded nucleic acid moiety does not contain a linking region and/or a flanking region described below and contains only one unmodified DNA region (103). That is, the whole region (101) of the single-stranded nucleic acid moiety corresponds to the unmodified DNA region (103).

In addition, the nucleic acid molecule of the present invention can optionally include a linking region (204), a flanking region (205), and/or a spacer region (207) shown in Figure 2. These regions are optional structural elements of the nucleic acid molecule of the present invention.

### (1-1) Unmodified DNA region

The "unmodified DNA region" (103, 203) is a nucleic acid region consisting of a nucleotide sequence completely or sufficiently complementary to the nucleotide sequence of a functional strand in a target RNAi molecule (100, 200). The unmodified DNA region is composed of DNA only.

Throughout the present specification, the term "unmodified DNA" refers to DNA that is not modified. The modification of nucleic acid is as described above.

Throughout the present specification, the term "functional strand" refers to a nucleic acid strand having a substantial RNAi activity that can induce gene silencing, in a target RNAi molecule, and is a nucleic acid strand that can be a real target of the nucleic acid molecule of the present invention. For example, when the RNAi molecule is siRNA or shRNA, the functional strand is the antisense strand called guide strand. When the RNAi molecule is miRNA, the functional strand is the antisense strand called miRNA strand.

Throughout the present specification, the term "complementary" refers to a relationship between two nucleotides allowing formation of a Watson-Crick base pair, specifically, the relationship between adenine and thymine or uracil, and the relationship between cytosine and guanine.

Throughout the present specification, the term "completely complementary" refers to a relationship between two nucleic acid strands that all nucleotides of the nucleotide sequence of one nucleic acid strand can form base pairs with all corresponding nucleotides of the nucleotide sequence of the other nucleic acid strand. Accordingly, in the nucleic acid molecule of the present invention, if the unmodified DNA region of the single-stranded nucleic acid moiety consists of a nucleotide sequence completely complementary to the nucleotide sequence of a functional strand of a target RNAi molecule, it means that all nucleotides of the nucleotide sequence of the unmodified DNA region can form base pairs with all nucleotides of the nucleotide sequence of the functional strand.

Throughout the present specification, the term "sufficiently complementary" refers to a relationship that not all nucleotides of at least one of two nucleic acid strands form base pairs with all corresponding nucleotides of the nucleotide sequence of the other nucleic acid strand and that 50% or more and less than 100%, preferably 60% or more and less than 100%, more preferably 70% or more and less than 100%, and most preferably 80% or more and less than 100% of the nucleotides of the nucleotide sequence of one of the nucleic acid strands can form base pairs with the nucleotides of the nucleotide sequence of the other nucleic acid strand. example, in two nucleic acid strands, the nucleotide sequence of one of the nucleic acid strands is not completely complementary, but only 50% or more and less than 100% complementary to the nucleotide sequence of one of the nucleic acid strand. Specific cases are, for example, a case that in two nucleic acid strands consisting of nucleotide sequences completely complementary to each other, only one of the nucleic acid strands has addition of one or more nucleotides and a case that in two nucleic acid strands, the nucleotide sequences of the two nucleic acid strands are not completely complementary to each other, but 50% or more and less than 100% of the nucleotide sequence of each nucleic acid strand is complementary to the nucleotide sequence of the other nucleic acid strand. Specific cases are, for example, a case that in two nucleic acid strands consisting of nucleotide sequences completely complementary to each other, both or either of bases of the nucleotide residues at a position corresponding to each other is substituted with other bases and thereby the nucleotide residues at the position of substitution cannot form a base pair and a case that one or two to four nucleotide residues are deleted from one of the nucleic acid strands and thereby the nucleotide residues at each position of deletion cannot form a base pair. In the nucleic acid molecule of the present invention, if the unmodified DNA region includes a mismatch site (206), it is an example of the sufficiently complementary strand.

Throughout the present specification, the term "mismatch site" (206) refers to a site consisting of nucleotide residues which cannot form a base pair, because a nucleotide complementary to the nucleotide contained in the nucleotide sequence of one of two nucleic acid strands is not present in the corresponding position of the nucleotide sequence of the other nucleic acid strand, when two nucleic acid strands are allowed to form base pairs.

The unmodified DNA regions can have, in their nucleotide sequences, a mismatch site (also referred to as "gap site") consisting of one nucleotide (which corresponds to one nucleotide residue, the same shall apply hereinafter) that does not form a base pair with a functional strand and/or at least one mismatch site (also referred to as "loop site") of 2 to 10, preferably 3 to 8, and more preferably 4 to 6 successive nucleotides. At least one unmodified DNA region comprises a mismatch site of 3 to 8 successive nucleotides that form a loop when the unmodified DNA region is allowed to form base pairs with the functional strand.

When the unmodified DNA region includes mismatch sites, the insert position is not particularly limited, as long as the insert position is within the unmodified DNA region, and is preferably inside of the unmodified DNA region, excluding both end parts of the same region, more preferably at the central portion of the unmodified DNA region, specifically, between the 9th to 14th or the 10th to 13th nucleotide residues from the 5'-end.

The length of the unmodified DNA region is not particularly limited, but the unmodified DNA preferably has a nucleotide length longer than the functional strand, from the viewpoint of having a nucleotide sequence that is completely or sufficiently complementary to the nucleotide sequence of a functional strand in a target RNAi molecule as described above. It is generally known that the functional strand of an RNAi molecule has a length of 18 to 25 nucleotides (Kim D.H., et al., 2005, Nat Biotechnol., 23(2): 222-6). A length of 18 to 35, 18 to 33, or 18 to 31 nucleotides is preferred considering that the unmodified DNA region includes a mismatch site described below.

### (1-2) Linking region

The "linking region" (204) is a nucleic acid region for mediating the link between the unmodified DNA region and the double-stranded nucleic acid moiety. The linking region is located at the 5'-end part and/or the 3'-end part of the single-stranded nucleic acid moiety. In the nucleic acid molecule of the present invention, at least one end part of the unmodified DNA region contained in the single-stranded nucleic acid moiety may be directly linked to the double-stranded nucleic acid moiety, but the mediation by the linking region increases the degrees of freedom of the single-stranded nucleic acid moiety and the double-stranded nucleic acid moiety to provide a higher effect of inhibiting the activity of an RNAi molecule.

The linking region is composed of a single strand having a length of 1 to 10, preferably 1 to 8, and more preferably 2 to 6 nucleotides.

The linking region may be composed of any nucleic acid described above and is preferably composed of natural nucleic acid and more preferably DNA. The nucleotide sequence of the linking region is not particularly limited, as long as the sequence does not form any conformation by intramolecular folding such as self-annealing. Examples of such nucleotide sequences include sequences composed of T only or C only.

### (1-3) Flanking region

The "flanking region" (205) is a single-stranded nucleic acid region in the single-stranded nucleic acid moiety (201) linked at the 5'-end or the 3'-end to which the double-stranded nucleic acid moiety is not linked. Accordingly, the flanking region is directly linked to one end part of the unmodified DNA region contained in the single-stranded nucleic acid moiety.

The nucleotide length of the flanking region is not particularly limited. The length is generally within a range of 1 to 30 nucleotides or preferably 1 to 25 nucleotides, considering that an unnecessarily long nucleotide strand makes it difficult to prepare the nucleic acid molecule of the present invention by, for example, chemical synthesis and also increases the cost.

The flanking region may be composed of any nucleic acid described above and is preferably composed of natural nucleic acid and more preferably DNA. The nucleotide sequence of the flanking region is not particularly limited, as long as the sequence does not form any conformation by intramolecular folding such as self-annealing. In addition, the flanking region can optionally include a nucleotide sequence encoding a wide variety of tag, such as a His tag, a FLAG tag, a myc tag, or a HA tag.

In the flanking region, the other end part not linked to the single-stranded nucleic acid moiety may be in a free state or may be immobilized to a carrier. Herein, examples of the "carrier" include low molecular compounds (e.g., biotin, avidin, streptavidin, and neutravidin), amino acids, peptides, macromolecular polysaccharide supports (e.g., sepharose, sephadex, and agarose), resins (natural resins and synthetic resins including plastics), silica, glass, magnetic beads, metals (e.g., gold, platinum, and silver), ceramics, and combinations thereof.

### (1-4) Spacer region

The "spacer region" (207) is a nucleic acid region for mediating two unmodified DNA regions among each unmodified DNA region, when the single-stranded nucleic acid moiety contains two or more unmodified DNA regions. In that case, the two or more unmodified DNA regions may be directly linked to each other, but the mediation by the spacer region increases the degrees of freedom of each unmodified DNA region in the single-stranded nucleic acid moiety, which allows each unmodified DNA regions to easily bind to the respective target RNAi molecules and thereby provides a higher effect of inhibiting RNAi molecule activity.

The spacer region is composed of a single strand having a length of 1 to 10, preferably 1 to 8, and more preferably 2 to 8 nucleotides.

The spacer region may be composed of any nucleic acid described above and is preferably composed of natural nucleic acid and more preferably DNA. The nucleotide sequence of the spacer region is not particularly limited, as long as the sequence does not form any conformation by intramolecular folding such as self-annealing.

### (2) Double-stranded nucleic acid moiety

The "double-stranded nucleic acid moiety" (102, 202) is a nucleic acid moiety linked to at least one of the 5'-end part and the 3'-end part of the single-stranded nucleic acid moiety.

### (2-1) Basic structure

The double-stranded nucleic acid moiety is composed of two nucleic acid strands complementary to each other forming base pairs. However, as described in paragraph (2-2) below, when a nucleic acid strand forms a double strand by intramolecular folding, can form a multiple strand structure, or has a nick in one or both of the nucleic acid strands, the double-stranded nucleic acid moiety may not be composed of two nucleic acid strands.

The nucleotide length of the each nucleic acid strand constituting the double-stranded nucleic acid moiety is not particularly limited, as long as the base pairs formed between the nucleic acid strands can stably maintain. In general, a length of 5 to 25 nucleotides is sufficient. However, the above length may be 26 nucleotides or more as necessary, considering that the preparation of a nucleic acid molecule having a long nucleotide length is difficult and expensive. In addition, teach nucleic acid strand constituting the double-stranded nucleic acid moiety may have the same or different nucleotide lengths, but preferably have the same nucleotide lengths. When each nucleic acid fragment has different nucleotide lengths, as shown in Figure 2B, the long strand side may form one or more side-chain stem sites (208) and a side-chain loop site (209) by intramolecular folding. The stem site may contain one or more side-chain mismatches/bulge sites (210).

The double-stranded nucleic acid moiety is preferably composed of two nucleic acid strands that are completely complementary to each other. In particular, nucleic acid strands each having a length of 9 nucleotides or less are desirably completely complementary to each other for stably maintaining the base pairs formed between the nucleic acid strands. On the other hand, nucleic acid strands each having a length of 10 nucleotides or more are not required to be completely complementary to each other and may be sufficiently complementary to each other. For example, at least one of the nucleic acid strands of the double-stranded nucleic acid moiety includes, in the nucleotide sequence, at least one mismatch site (211) of one and/or two or more successive nucleotides that do not form base pairs with the other nucleic acid strand. When the mismatch site forms a loop site (212) composed of two or more successive nucleotides, the nucleotide length of the mismatch site may be 2 to 6 nucleotides. When the mismatch site forms one or more loop structures and one or more stem structures by intramolecular folding in the loop site, as described above, the mismatch site may have a longer nucleotide length.

The double-stranded nucleic acid moiety may be composed of any nucleic acid described above. The nucleic acid is not particularly limited, as long as the nucleic acid can form a stable conformation as in a secondary structure such as a double-strand structure or a tertiary structure such as a G-quartet described below, and is preferably composed of DNA in consideration of the synthesis and the cost of the nucleic acid molecule of the present invention. That is, the nucleic acid molecule of the present invention wholly composed of DNA can be most easily and inexpensively and is therefore preferred from the viewpoint of synthesis and cost of the nucleic acid molecule.

Each nucleic acid strand of the double-stranded nucleic acid moiety may have any nucleotide sequence as long as the nucleic acid strands are completely or sufficiently complementary to each other, as described above. In addition, one or both of the nucleic acid strands may have a nucleotide sequence that can form a conformation by intramolecular folding.

In the double-stranded nucleic acid moiety, at least one nucleic acid strand is linked to a single-stranded nucleic acid moiety at least one end. Accordingly, when each end of each nucleic acid strand is linked to a single-stranded nucleic acid moiety, one double-stranded nucleic acid moiety can be linked to four single-stranded nucleic acid moieties at most.

### (2-2) Double-stranded nucleic acid moiety having specific structure

The double-stranded nucleic acid moiety is in principle composed of two nucleic acid strands completely or sufficiently complementary to each other to form base pairs. However, when a nucleic acid strand forms a double strand by intramolecular folding, can form a multiple strand structure, or has a nick in one or both of the two nucleic acid strands complementary to each other, the double-stranded nucleic acid moiety can be formed of one or three or more nucleic acid strands. Such specific structures are also the same or similar structure with the double-stranded nucleic acid formed by base pairs between two nucleic acid strands in the point that a conformation is formed through base pairing between nucleic acid strands or in a nucleic acid strand. Accordingly, in the nucleic acid molecule of the present invention, such specific structures are also included in the double-stranded nucleic acid moiety.

Specific examples of the double-stranded nucleic acid moiety having such a specific structure are shown in Figures 3-1 and 3-2.

Diagram A in Figure 3-1 shows a structural example in which two nucleic acid strands (301) complementary to each other are linked by a loop region (302). In this structure, a single nucleic acid strand forms the double-stranded nucleic acid moiety with the loop region.

Throughout the present specification, the term "loop region" (302) refers to, in two nucleic acid strands completely or sufficiently complementary to each other constituting the double-stranded nucleic acid moiety, a nucleic acid region composed of a single strand that links the 3'-end of one nucleic acid strand to the 5'-end of the other nucleic acid strand. The nucleotide length of the loop region is not particularly limited, and the length may be generally 3 to 10 nucleotides. In a case that the double-stranded nucleic acid moiety is composed of a single nucleic acid strand, the essential structural unit of the nucleic acid molecule of the present invention can be formed from the single nucleic acid strand by linking the single-stranded nucleic acid moiety (304) to one end of the double-stranded nucleic acid moiety. Accordingly, the nucleic acid molecule of the present invention can be produced merely by chemically synthesizing a nucleic acid molecule and then causing intramolecular folding of the molecule, and no another step, such as adjustment of mixing ratio of different two nucleic acid strands, is required, resulting in reduction in the manufacturing step and manufacturing cost.

Diagram B in Figure 3-1 shows a structural example of the double-stranded nucleic acid moiety in which a triplex (triple helix structure) is formed between nucleic acid strands. The triplex is formed between three nucleic acid strands each having a specific nucleotide sequence. Examples of the "specific nucleotide sequence" include a sequence (GA sequence) consisting of successively sequenced G (guanine) and A (adenine) and a sequence (TC sequence) consisting of successively sequenced T (tymine) and C (cytosine). When two of three nucleic acid strands contain GA sequences and one strand contains a CT sequence, as shown in diagram B, base pairs are formed among three nucleotides, that is, among GGC (each nucleotides is present apart from one another on the nucleotide sequence) and among AAT (the same as above).

Diagram C in Figure 3-1 shows a structural example of the double-stranded nucleic acid moiety in which a quadruplex (quadruple helix structure) is formed between nucleic acid strands. The quadruplex shown in this diagram is a structure (306) called G-quartet, wherein four Gs lie in the same plane through hydrogen bonds and that two or more of such planes are formed. The G-quartet can be formed in a single strand containing a G-rich nucleotide sequence, such as that set forth in SEQ ID NO: 41 (305), by intramolecular folding.

Diagrams D and E in Figure 3-2 show structural examples of the double-stranded nucleic acid moiety (303) having a nick (307) in one (D) or both (E) of two nucleic acid strands complementary to each other and forming base pairs. In these structures, the double-stranded nucleic acid moiety is composed of three or four or more nucleic acid strands.

When one of the nucleic acid strands has a nick at one position (D), the nucleic acid strand is divided into two strands (308, 309) by the nick. Accordingly, the double-stranded nucleic acid moiety has a structure in which two nucleic acid strands are base-paired with one nucleic acid strand. These nucleic acid strands can form a double-stranded nucleic acid moiety composed of two nucleic acid strands as a whole through base pairing.

When both of the nucleic acid strands each have a nick at one position (E), the two nucleic acid strands are each divided into two strands by the nick. Accordingly, the double-stranded nucleic acid moiety as a whole has a structure in which four nucleic acid strands (308, 309, 310, 311) form base pairs and thereby form a double-stranded nucleic acid moiety composed of two nucleic acid strands as a whole. In order to avoid that the double-stranded nucleic acid moiety is divided into two parts, the positions of the nicks in each nucleic acid strand must not correspond to each other in annealing. In order to form a stable double-stranded nucleic acid moiety, the positions of nicks in each nucleic acid strand are apart from each other by 4 or more nucleotides, preferably 6 or more nucleotides, and more preferably 8 or more nucleotides, when the annealing of each strand is performed.

A plurality of nicks may be present per single nucleic acid strand in the two nucleic acid strands complementary to each other in principle of the structure. However, in such a structure, the nucleic acid strands constituting the double-stranded nucleic acid moiety are fractionated, resulting in complication of the process for producing the nucleic acid molecule of the present invention. Accordingly, the number of the nicks per one nucleic acid strand is preferably one.

### 1-3-2. Structural embodiment of the nucleic acid molecule for inhibiting the activity of an RNAi molecule

The nucleic acid molecule of the present invention has at least one minimum and indispensable structural unit, in one molecule, consisting of one single-stranded nucleic acid moiety and one double-stranded nucleic acid moiety linked to each other. In contrast, the nucleic acid molecule of the present invention can have two or more single-stranded nucleic acid moieties and/or double-stranded nucleic acid moieties in one molecule.

In this case, the structures of each single-stranded nucleic acid moiety included in one molecule may be the same or different or may be a combination thereof (when the number of the single-stranded nucleic acid moieties is three or more).

For example, when the single-stranded nucleic acid moieties each include one unmodified DNA region, the target RNAi molecules of respective single-stranded nucleic acid moieties may be the same or different or may be a combination thereof (only when the number of the single-stranded nucleic acid moieties is three or more). When the target RNAi molecules of each single-stranded nucleic acid moiety are the same, such nucleic acid molecule can inhibit a larger amount of target RNAi molecules with one molecule and is therefore effective. When the target RNAi molecules of each single-stranded nucleic acid moiety are different from one another, each single-stranded nucleic acid moiety can target respective RNAi molecules. Accordingly, a multifaceted therapeutic effect by one nucleic acid molecule can be expected, and such nucleic acid molecule is therefore effective when the disease to be treated is caused by the activity of a plurality of types of miRNA *in vivo.*

In addition, each single-stranded nucleic acid moiety may have the same or different structures. For example, even if the targets are the same RNAi molecules, one single-stranded nucleic acid molecule is composed of an unmodified DNA region only, and the unmodified DNA region consists of a nucleotide sequence completely complementary to the nucleotide sequence of a functional strand of a target RNAi molecule; and the other one-stranded nucleic acid molecule is composed of an unmodified DNA region, a linking region, and a flanking region, and the unmodified DNA region consists of a nucleotide sequence having a mismatch site and being sufficiently complementary to the nucleotide sequence of the functional strand of the target RNAi molecule. The strength of the effect of inhibiting the activity of a target RNAi by a single-stranded nucleic acid moiety can vary depending on, for example, the presence and the position of a mismatch site or the presence of a linking region. Accordingly, the structural difference in each single-stranded nucleic acid moiety described above is useful, for example, when the target RNAi molecules of each single-stranded nucleic acid moiety are different and when the strengths of inhibiting the activities of each target RNAi are required to be different.

Alternatively, each double-stranded nucleic acid moiety may have structures different from one another. For example, in one molecule, one double-stranded nucleic acid moiety is composed of two nucleic acid strands, and the other double-stranded nucleic acid moiety is composed of a single strand having a loop region. In another example, when two or more double-stranded nucleic acid moieties are each composed of two nucleic acid strands, one double-stranded nucleic acid moiety is composed of two nucleic acid strands completely complementary to each other, and the other double-stranded nucleic acid moiety is composed of nucleotide sequences having a mismatch site and being sufficiently complementary to each other.

When the structures of each single-stranded nucleic acid moiety are the same or different, the number of each unmodified DNA region in each single-stranded nucleic acid moiety may be the same or different or may be a combination thereof (only when the number of the single-stranded nucleic acid moieties is three or more). Regardless of the number of the unmodified DNA regions, the target RNAi molecules of the respective unmodified DNA regions may be the same or different or may be a combination thereof (only when the number of the unmodified DNA region is three or more).

In addition, when one molecule includes two or more double-stranded nucleic acid moieties, the structure of each double-stranded nucleic acid moiety may be the same or different or may be a combination thereof (only when the number of the double-stranded nucleic acid moieties is three or more).

Various structural embodiments of the nucleic acid molecule of the present invention having a plurality of single-stranded nucleic acid moieties and/or double-stranded nucleic acid moieties in one molecule will now be described with reference to Figures 4 to 6.

### (1) Nucleic acid molecule comprising one double-stranded nucleic acid moiety and two to four single-stranded nucleic acid moieties in one molecule (Figure 4)

When the double-stranded nucleic acid moiety is composed of two nucleic acid strands, the double-stranded nucleic acid moiety has two 5'-ends and two 3'-ends. Accordingly, one double-stranded nucleic acid moiety can have two (A-1 to A-7 in Figure 4), three (B-1 to B-3 in Figure 4), or four (C in Figure 4) single-stranded nucleic acid moieties.

In addition, when the double-stranded nucleic acid moiety is composed of one nucleic acid strand, the double-stranded nucleic acid moiety has one 5'-end and one 3'-end. Accordingly, one double-stranded nucleic acid moiety can have two single-stranded nucleic acid moieties (D in Figure 4).

### (2) Nucleic acid molecule including two double-stranded nucleic acid moieties and one to seven single-stranded nucleic acid moieties in one molecule (Figures 5-1 to 5-11)

When two double-stranded nucleic acid moieties are each composed of two nucleic acid strands, one double-stranded nucleic acid moiety can have one single-stranded nucleic acid moiety (Figure 5-1: A) or two (Figure 5-1: B-1 to B-7), three (Figures 5-2 and 5-3: C-1 to C-19), four (Figures 5-4 to 5-6: D-1 to D-24 and other nucleic acid molecules (not shown) having structures, in each of which a loop-formed single strand nucleic acid moiety is linked to the corresponding 5'-end and 3'-end of the double-stranded nucleic acid moiety), five (Figure 5-7: E-1 to E-10 and other nucleic acid molecules (not shown) having structures, in each of which a loop-formed single strand nucleic acid moiety is linked to the corresponding 5'-end and 3'-end of the double-stranded nucleic acid moiety), six (Figure 5-8: F), or seven (Figure 5-8: G) single-stranded nucleic acid moieties.

When one double-stranded nucleic acid moiety is composed of one single nucleic acid strand and the other double-stranded nucleic acid moiety is composed of two nucleic acid strands, one double-stranded nucleic acid moiety can have one single-stranded nucleic acid moiety (Figure 5-9: H) or two (Figure 5-9: 1-1 to 1-2 and other nucleic acid molecules (not shown) having structures, in each of which a loop-formed single strand nucleic acid moiety is linked to the corresponding 5'-end and 3'-end of the double-stranded nucleic acid moiety), three (Figure 5-9: J-1 to J-9 and other nucleic acid molecules (not shown) having structures, in each of which a loop-formed single strand nucleic acid moiety is linked to the corresponding 5'-end and 3'-end of the double-stranded nucleic acid moiety), four (Figure 5-10: K-1 to K-7 and other nucleic acid molecules (not shown) having structures, in each of which a loop-formed single strand nucleic acid moiety is linked to the corresponding 5'-end and 3'-end of the double-stranded nucleic acid moiety), or five (Figure 5-10: L) single-stranded nucleic acid moieties.

When both of the two double-stranded nucleic acid moieties are composed of one nucleic acid strand, one of the double-stranded nucleic acid moieties can have one (Figure 5-11: M) or two (Figure 5-11: N-1 to N-2) or three (Figure 5-11: O-1 to 0-3) single-stranded nucleic acid moieties.

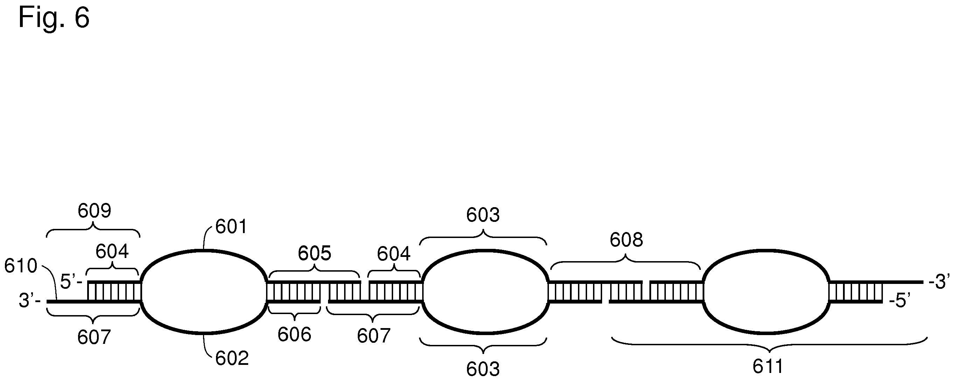

### (3) Nucleic acid molecule including three or more double-stranded nucleic acid moieties and three or more single-stranded nucleic acid moieties in one molecule (Figure 6)

The nucleic acid molecule of the present invention can encompass three or more double-stranded nucleic acid moieties and three or more single-stranded nucleic acid moieties in one molecule. However, in many of such nucleic acid molecules, the production process involves complicated annealing of a plurality of different nucleic acid fragments or synthesis of a very long nucleic acid strand having a length exceeding 150 nucleotides, resulting in an increase in the number of production process, an increase in manufacturing cost, and also a difficulty in the production itself. Thus, such nucleic acid molecules are against the purpose of the present invention and it is hard to say that such a structure is preferred.

However, for example, when a nucleic acid molecule encompassing a large number of double-stranded nucleic acid moieties and single-stranded nucleic acid moieties can be produced by mixing and annealing two or more different nucleic acid strands each having a length of about 40 to 60 nucleotides, the nucleic acid molecule is free from the above-mentioned problems and is therefore excluded from the above-mentioned definition.

For example, the nucleic acid molecule shown in Figure 6 is composed of two different nucleic acid strands (here, conveniently, referred to as "α-strand (601)" and "β-strand" (602)) in which both ends of each single-stranded nucleic acid moiety are each linked to a part of one nucleic acid strand of each double-stranded nucleic acid moiety. In the α-strand, a part (604) of one nucleic acid strand of the double-stranded nucleic acid moiety lying at the 5'-end side of a single-stranded nucleic acid moiety (603) is base-paired with a part (607) of the other nucleic acid strand of the double-stranded nucleic acid moiety lying at the 3'-end of the single-stranded nucleic acid moiety (603) in the β-strand, and thereby a part (609) of a double-stranded nucleic acid fragment (608) having a cohesive end (610) is formed. Similarly, in the α-strand, a part (605) of one nucleic acid strand of the double-stranded nucleic acid moiety lying at the 3'-end side of a single-stranded nucleic acid moiety is base-paired with a part (606) of the other nucleic acid strand of the double-stranded nucleic acid moiety lying at the 5'-end of the single-stranded nucleic acid moiety in the β-strand, and thereby a part of a double-stranded nucleic acid fragment having a cohesive end is formed. The nucleic acid molecule of the present invention has a structure in which a plurality of partial structural units (611) of the nucleic acid molecule of the present invention each consisting of the thus-formed two single-stranded nucleic acid moieties and parts of double-stranded nucleic acid fragments linked to both ends of each single-stranded nucleic acid moiety and having cohesive ends are linked to one another with the cohesive ends.

### 1-4. Method of producing the nucleic acid molecule for inhibiting the activity of an RNAi molecule

A method of preparing the nucleic acid molecule of the present invention will be described. The nucleic acid molecule of the present invention can be produced by any method known in the art that can prepare a nucleic acid molecule having a structure described in "1-3. Structure" above. Herein, one specific example will now be described, but the method of producing the nucleic acid molecule of the present invention is not limited to the following method.

The method of producing the nucleic acid molecule of the present invention comprises (1) designing step, (2) synthetic step, and (3) annealing step.

### (1) Designing step

The "designing step" determines a structure of the nucleic acid molecule of the present invention and a nucleotide sequence constituting the structure.

In this step, first, the single-stranded nucleic acid moiety is designed. In the designing of the single-stranded nucleic acid moiety, based on the nucleotide sequence of a functional strand in an target RNAi molecule of interest, a nucleotide sequence complementary to the nucleotide sequence is determined and is then designed as an unmodified DNA region. On this occasion, a mismatch site is inserted in the unmodified DNA region as necessary. In addition, when a nucleic acid molecule comprising two or more unmodified DNA regions for different target RNAi molecules is produced, unmodified DNA regions specific to the respective RNAi molecules are designed. If necessary, in the unmodified DNA region, the end part to which a double-stranded nucleic acid moiety is linked may be designed to place a linking region, or the end part to which no double-stranded nucleic acid moiety is linked may be designed a flanking region. When a single-stranded nucleic acid moiety comprises two or more unmodified DNA regions, a spacer region may be designed to place between the nucleotide sequences of the unmodified DNA regions. The nucleotide sequences and the nucleotide lengths of the linking region, flanking region, and spacer region may be appropriately determined depending on the necessity and the lengths of polynucleotides to be synthesized. The linking region, flanking region, and spacer region can be designed so as to be composed of nucleic acid other than DNA, if necessary.

Next, the double-stranded nucleic acid moiety is designed. Herein, first, whether the double-stranded nucleic acid moiety is composed of two nucleic acid strands or is composed of one nucleic acid strand is determined. In the case composed of two nucleic acid strands, each nucleic acid strands are designed to be completely or sufficiently complementary to each other. On this occasion, the nucleotide sequence of each nucleic acid strand must be designed not to cause self-annealing or not to anneal with a single-stranded nucleic acid moiety. In the case composed of one nucleic acid strand, completely or sufficiently complementary nucleic acid strands are designed such that the 3'-end of one of the nucleic acid strands and the 5'-end of the other nucleic acid strand are linked with a loop region to provide a single nucleic acid strand. The nucleic acid constituting the double-stranded nucleic acid moiety may be natural nucleic acid, artificial nucleic acid, or a combination thereof. In the designing, the nucleic acid is appropriately selected as necessary and, generally, is preferably DNA.

Subsequently, it is designed such that at least one end of a single-stranded nucleic acid moiety and at least one end of a double-stranded nucleic acid moiety are linked to provide a single nucleic acid strand. This linking is not necessarily performed in the designing step and may be performed in the subsequent synthetic step.

### (2) Synthetic step

The "synthetic step" produces, by enzymatic or chemical synthesis, each nucleic acid strand constituting the nucleic acid molecule of the present invention based on the nucleotide sequence designed in the designing step. As described above, the nucleic acid molecule of the present invention excluding the unmodified DNA region can be composed of natural nucleic acid, artificial nucleic acid, or a combination thereof. The nucleic acid may be synthesized by any technology known in the art, for example, can be chemically synthesized in accordance with a solid phase synthesis. Specifically, for example, the chemical synthesis described in Current Protocols in Nucleic Acid Chemistry, Volume 1, Section 3, Verma S. and Eckstein F., 1998, Annul Rev. Biochem., 67, 99-134 can be employed. Many life science manufacturers (e.g., Takara Bio Inc., FASMAC Co., Life Technologies, Gene Design Inc., and Sigma-Aldrich Corporation) provide contract manufacturing services, which may be also used for chemical synthesis of nucleic acid such as artificial nucleic acid or modified nucleic acid. The nucleic acid molecule of the present invention after chemical synthesis is preferably purified prior to the use by a method known in the art. Examples of the purification include gel purification, affinity column purification, and HPLC.

When the single-stranded nucleic acid moiety and the double-stranded nucleic acid moiety are separately chemically synthesized, as described above, the nucleic acid molecule of the present invention is synthesized in this step. For example, the nucleic acid molecule may be synthesized by linking both ends of a single nucleic acid fragment by a method known in the art. In one specific example of such a method, an enzyme such as ligase is used.

### (3) Annealing step

The "annealing step" forms the nucleic acid molecule of the present invention having an activity of inhibiting the activity of a target RNAi molecule by performing annealing and/or intramolecular folding of each nucleic acid strand constituting the nucleic acid molecule after the synthetic step.

This step can be achieved by dissolving the synthesized one or more nucleic acid strands in, for example, an appropriate buffer, e.g., D-PBS(-) (0.2 g/L KCl, 8 g/L NaCl, 0.2 g/L KH2PO4, 1.15 g/L Na2HPO4), mixing the solution, heating the solution to 90°C, and then gradually cooling the solution to cause annealing.

When the nucleic acid molecule of the present invention is composed of two or more nucleic acid strands, the nucleic acid molecule can be produced by chemically synthesizing the respective nucleic acid strands independently, purifying the nucleic acid strands as necessary, and then mixing the nucleic acid strands, preferably, at the same quantities to cause annealing with each other.

When the nucleic acid molecule of the present invention is composed of a single nucleic acid strand, the nucleic acid molecule can be produced by chemically synthesizing the nucleic acid strand, purifying the nucleic acid strand as necessary, and then exposing the nucleic acid strand to conditions allowing intramolecular folding.

### 1-5. Using method

The nucleic acid molecule of the present invention can inhibit the activity of a target RNAi molecule *in vivo* by being introduced into a living body, i.e., a living cell, tissue, organ, or individual.

Examples of the method of introducing the nucleic acid molecule of the present invention into a living body include injection of a solution containing the nucleic acid molecule of the present invention, bombardment with particles coated with the nucleic acid molecule of the present invention, and electroporation in the presence of the nucleic acid molecule of the present invention. Another method for introducing a nucleic acid into a cell, known in the field of the art, such as lipid-mediated carrier transport or chemical-mediated transport (e.g., calcium phosphate transfection), can be employed. When the living body is an animal individual, the nucleic acid molecule can be introduced as a drug such as a pharmaceutical composition described below.

The nucleic acid molecule of the present invention may be introduced into any cell, tissue, organ, or individual without particular limitation. Examples thereof include biological species that can encompass a target RNAi molecule; and cells, tissues, and organs derived from such biological species. The biological species may be any organism such as an animal and plant.

The animal is preferably a vertebrate animal and more preferably fish, bird, or mammal. Further preferred examples of the fish include fishery resources (e.g., fish species of Salmonidae, Serranidae, Gadidae, Clupeidae, Paralichthyidae, Pleuronectidae, Carangidae, Ammodytidae, Sparidae, and Sebastidae). Further preferred examples of the bird include edible species (e.g., chicken, goose, duck, wild duck, domestic duck, turkey, quail, and ostrich). Further preferred examples of the mammal include livestocks (pig, bovine, sheep, goat, and horse), experimental animals (rodents, rabbit, dog, and monkey), racehorses, pets (dog, cat, rabbit, monkey, and rodents), and humans. Most preferred biological species is a human.

On the other hand, when the biological species is a plant, the plant is preferably a seed plant, more preferably an angiosperm, and further preferably an edible plant species, a plant species for fiber resources, or a plant species for wood resources. Examples of the edible plant species include Gramineae (e.g., rice, wheat, barley, rye, corn, kaoliang, and millet), Fabaceae (e.g., soybean, azuki, and green pea), Solanaceae (e.g., tomato, eggplant, potato, red pepper, and green pepper), Convolvulaceae (e.g., sweet potato), Rosaceae (e.g., strawberry, almond, peach, pram, Japanese apricot, rose, and cherry), Brassicaceae (e.g., radish, turnip, and rape), Chenopodiaceae (e.g., spinach and sugar beet), and edible plant species belonging to Apiaceae, Polygonaceae, Cucurbitaceae, Asteraceae, Liliaceae, Araceae, Vitaceae, Rutaceae, Fagaceae, or Palmae. Examples of the plant species for fiber resources include cotton and hemp. Examples of the plant species for wood resources include Japanese cedar, Japanese cypress, fir, hemlock fir, pine, yew, cherry, maple, oak, Japanese oak, beech, elm, zelkova, walnut, magnolia, Japanese Judas tree, teak, lauan, ebony, mahogany, poplar, and eucalyptus.

The nucleic acid molecule of the present invention can also be used as a reagent for research, for example, as a gene expression inhibitor or an RNAi reagent.

The nucleic acid molecule of the present invention suppresses a specific RNAi molecule in a cell, tissue, organ, or individual according to the dose introduced thereinto. The nucleic acid molecule can be introduced in an amount that allows the delivery of at least one copy per cell. A high dose (for example, at least 5, 10, 100, 500, or 1000 copies per cell) of the nucleic acid molecule can more effectively cause suppression.

### 1-6. Effect

The nucleic acid molecule of the present invention can specifically and efficiently inhibit the activity of a target RNAi molecule. The nucleic acid molecule of the present invention, even if it is composed of DNA only, can sufficiently inhibit the activity of a target RNAi molecule and therefore can be chemically synthesized simply, in a large quantity, and inexpensively.

### 2. Pharmaceutical composition

### 2-1. Summary

A second embodiment of the present invention is a pharmaceutical composition. The pharmaceutical composition of the present invention can specifically inhibit the activity of a specific RNAi molecule *in vivo* by being administered into a living body.

### 2-2. Structure

### 2-2-1. Composition

### (1) Active ingredient

The pharmaceutical composition of the present invention contains the nucleic acid molecule for inhibiting the activity of an RNAi molecule of the first embodiment as an active ingredient. The pharmaceutical composition of the present invention can contain same or different two or more nucleic acid molecules against one target RNAi molecule. Alternatively, the pharmaceutical composition may contain same or different two or more nucleic acid molecules against different target RNAi molecules.

In the pharmaceutical composition of the present invention, the content of the nucleic acid molecule as an active ingredient of the pharmaceutical composition may be a pharmaceutically effective amount. Throughout the present specification, the term "pharmaceutically effective amount" refers to a dose necessary for that the functional nucleic acid in a nucleic acid molecule can exert the function and hardly or does not cause harmful side effects to the living body to which the pharmaceutical composition is administered. The specific dose varies depending on the type of the nucleic acid molecule to be used, the type of the target RNAi molecule, the mechanism of action of the RNAi molecule, the effects and stability of the nucleic acid molecule of the present invention, the dosage form of the pharmaceutical composition to be used, the type of the carrier to be used, the administrating method, the information of a subject, and the administration route. In the case of administration to a human, the range of the pharmaceutically effective amount and a preferred administration route are decided based on data generally obtained by cell culture assay and animal experiments. The ultimate dose for individual subject is determined by doctor's judgment and is adjusted. Examples of the information of the subject to be considered on this occasion include the progress or severity of the disease, systemic conditions of the subject's health, age, weight, sex, dietary habit, drug sensitivity, and tolerance to the therapy.

As a specific example of the content of the nucleic acid molecule per dosage unit, when the pharmaceutical composition of the present invention is injected into a human male adult (weight: 60 kg) not requiring being used together with another medicine, the nucleic acid molecule of the present invention may be contained about 0.01% to about 20% (w/v), preferably about 0.1% to about 10% (w/v) per dosage unit of an injection. If a large amount of the nucleic acid molecule of the present invention is required to be administered for obtaining the pharmacological effect of the pharmaceutical composition of the present invention, the pharmaceutical composition may be administered by dividing for several times for reducing the burden on a subject.

### (2) Vehicle

The pharmaceutical composition of the present invention can contain a vehicle as an active ingredient, i.e., the nucleic acid molecule described in the first embodiment. Examples of the vehicle include solvents such as water, ethanol, propylene glycol, ethoxylated isostearyl alcohol, polyoxylated isostearyl alcohol, and polyoxyethylene sorbitan fatty acid esters. These vehicles are desirably sterilized and are preferably adjusted to be isotonic with blood as necessary.

### (3) Carrier

The pharmaceutical composition of the present invention can contain a pharmaceutically acceptable carrier as necessary. The term "pharmaceutically acceptable carrier" refers to an additive that is generally used in the pharmaceutical technical field. Examples of the carrier include excipients, binders, disintegrants, fillers, emulsifiers, fluidity additive modifiers, and lubricants.

Examples of the excipient include sugars such as monosaccharides, disaccharides, cyclodextrins, and polysaccharides (more specific non-limiting examples thereof include glucose, sucrose, lactose, raffinose, mannitol, sorbitol, inositol, dextrin, maltodextrin, starch, and cellulose), metal salts (e.g., sodium chloride, sodium phosphate or calcium phosphate, calcium sulfate, magnesium sulfate, and calcium carbonate), citric acid, tartaric acid, glycine, low-, medium-, and high-molecular weight polyethylene glycols (PEGs), Pluronic, kaolin, silicic acid, and combinations thereof.

Examples of the binder include starch pastes from corn, wheat, rice, or potato starch, simple syrup, glucose solution, gelatin, tragacanth, methylcellulose, hydroxypropyl methylcellulose, sodium carboxymethyl cellulose, shellac, and polyvinyl pyrrolidone.

Examples of the disintegrant include the starches, lactose, carboxymethyl starch, crosslinked polyvinyl pyrrolidone, agar, laminarin powder, sodium bicarbonate, calcium carbonate, alginic acid or sodium alginate, polyoxyethylene sorbitan fatty acid esters, sodium lauryl sulfate, stearic acid monoglyceride, and salts thereof.

Examples of the filler include the sugars and calcium phosphates (e.g., tricalcium phosphate and calcium hydrogen phosphate).

Examples of the emulsifier include sorbitan fatty acid esters, glycerin fatty acid esters, sucrose fatty acid esters, and propylene glycol fatty acid esters.

Examples of the fluidity additive modifier and lubricant include silicates, talc, stearates, and polyethylene glycol.

These carriers are mainly used for facilitating the formulation of the dosage form, maintaining the dosage form and pharmaceutical effects, and preventing the nucleic acid molecule as an active ingredient from being degraded by nucleolytic enzymes *in vivo* and may be appropriately used as necessary. If necessary, the composition may further contain a flavoring agent, a solubilizer, a suspending agent, a diluent, a surfactant, a stabilizer, an absorption promoter, an extender, a humidity agent, a moisturizing agent, an adsorbent, a disintegration inhibitor, a coating agent, a coloring agent, a preservative, an antioxidant, a flavor, a flavoring agent, a sweetener, and a buffer, in addition to the above additives.

### (4) Other active ingredient

The pharmaceutical composition of the present invention may contain another active ingredient within a range that the pharmaceutical composition does not impair the pharmacological effect of the nucleic acid molecule as an active ingredient, i.e., may be a combined formulation. The "another active ingredient" is, for example, a drug inhibiting the activity of the same target RNAi molecule as that of the nucleic acid molecule in the first embodiment by a mechanism of action different from that of the nucleic acid molecule of the first embodiment. Such a combined formulation can inhibit the activity of the same target RNAi molecule from multiple aspects. Therefore, a synergistic effect can be expected.

In addition, the another active ingredient may be a drug having a pharmacological action different from that of the nucleic acid molecule of the first embodiment, such as an antibiotic.

### 2-2-2. Dosage form

The dosage form of the pharmaceutical composition of the embodiment is not particularly limited, as long as it has a dosage form that does not inactivate the nucleic acid molecule as an active ingredient or another additive active ingredient and that can exert the pharmacological effect *in vivo* after administration. In general, natural nucleic acid is easily degraded by nucleolytic enzymes, such as nucleases, *in vivo.* In administration of the pharmaceutical composition of the present invention, the nucleic acid molecule as an active ingredient is preferably in a dosage form that is hardly degraded *in vivo.* The dosage form may be, for example, a liquid, solid, or semisolid. Specific examples of the dosage form include parenteral dosage forms such as injections, suspensions, emulsions, ophthalmic solutions, nasal drops, creams, ointments, plasters, fomentations, and suppositories; and oral dosage forms such as liquids, powders, granules, tablets, capsules, sublingual formulations, and troches. The dosage form is preferably an injection.

The pharmaceutical composition of the embodiment may be prepared in a form of nanoparticles (including, for example, the target nanoparticle-delivering system described in Davis M.E., et al., Nature, 2010, 464: 1067-1070), liposome (including, for example, membrane-permeable peptide binding liposomes, SNALPs), or a cholesterol binder. The RNAi-delivering system described in Castanotto D. & Rossi J.J., Nature, 2009, 457, 426-433 can also be used.

### 2-3. Production of pharmaceutical composition

The pharmaceutical composition of the present invention may be produced by a formulation method known to those skilled in the art. For example, the method described in Remington's Pharmaceutical Sciences (Merck Publishing Co., Easton, Pa.) can be referred to.

### 2-4. Route of administration

The pharmaceutical composition of the present embodiment allows the nucleic acid molecule in the first embodiment as an active ingredient to be administered to a living body in an amount pharmaceutically effective for treating a disease of interest. The living body to which the composition is administered is a vertebrate animal, preferably a mammal, and more preferably a human.

The administration method of the pharmaceutical composition of the present invention may be systemic administration or topical administration, which can be appropriately selected depending on, for example, the type of a disease, the site of onset, or the progress of the disease. When the site of disease onset is local, direct topical administration by, for example, injection to the onset site and the circumference thereof is preferred. Such administration allows a sufficient amount of the nucleic acid molecule as an active ingredient to be administered to the site (tissue or organ) to be treated and also hardly affects other tissues. In contrast, in the cases of being incapable of specifying the site to be treated, as in metastatic cancer, or of systemic onset of a disease, systemic administration by, for example, intravenous injection is preferred. Such administration allows the nucleic acid molecule as an active ingredient to be distributed via the blood flow throughout the body, resulting in a possibility of administration to a lesion area that cannot be found by diagnosis.

As an specific administration method, the pharmaceutical composition of the present invention can be administered by any method that does not inactivate the nucleic acid molecule as an active ingredient. For example, the administration may be parenteral (e.g., injection, aerosol, application, instillation, or rhinenchysis) or oral and is particularly preferably injection because of the above-described reasons and the relatively low invasiveness.

In administration by injection, the injection site is not particularly limited, as long as the nucleic acid molecule of the present invention or the nucleic acid molecule produced from an expression vector exert its function on a target molecule and can achieve the purpose of the pharmaceutical composition. Examples of the injection site include veins, arteries, the liver, muscles, joints, the marrow, the medullary cavity, the ventricles, transcutaneous sites, subcutaneous sites, intracutaneous sites, the abdominal cavity, the nasal cavity, the bowels, and sublingual sites. Preferred injection sites are blood vessels, such as intravenous injection and intraarterial injection.

### Examples

The present invention will now be more specifically described by examples. However, the technical scope of the present invention is not limited to the following examples.

### <Example 1: Preparation of the nucleic acid molecule for inhibiting the activity of miR-16 and verification of its effect of inhibiting the activity (1)>

### (Purpose)

The nucleic acid molecules of the present invention in various structural embodiments of which the target RNAi molecule is miR-16 as endogenous miRNA of HEK293T cells (human embryonic kidney cell line) were constructed and verified for the effect of inhibiting the miR-16 activity.

### (Method)

The effect of inhibiting the miR-16 activity was calculated as an increase in rate of expression of fluorescent protein when the nucleic acid molecule for inhibiting the activity of miR-16 of the present invention was added to a system of cells in which the translation of the fluorescent protein was inhibited by miR-16. A specific method of implementing the Example will now be described.

### 1. Preparation of nucleic acid molecule

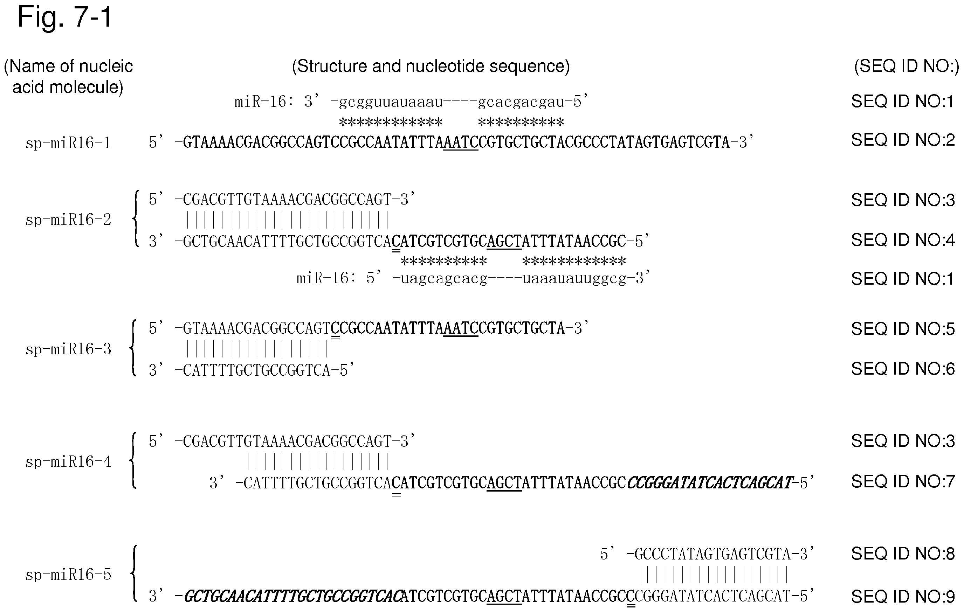

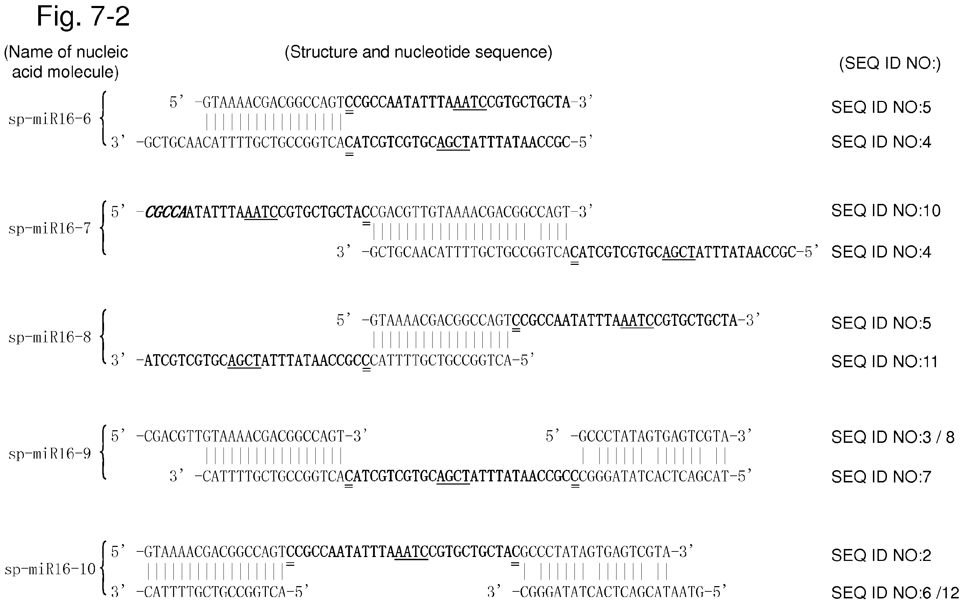

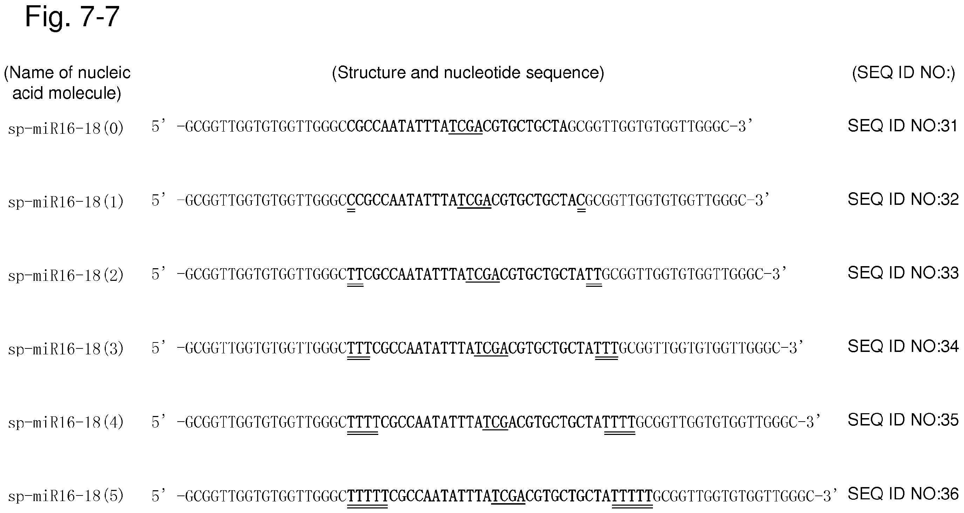

Figures 7-1 to 7-7 show the names, structures, and nucleotide sequences of the nucleic acid molecules used in the Example. The nucleic acid strand consisting of DNA oligonucleotides or RNA oligonucleotides constituting each nucleic acid molecule was chemically synthesized. DNA oligonucleotides were synthesized by FASMAC Co., Ltd. under contract. RNA oligonucleotides were synthesized by FASMAC Co., Ltd. or Sigma-Aldrich Japan K.K. under contract. Each nucleic acid was not modified. The synthesized oligonucleotides were each dissolved in D-PBS(-) (0.2 g/L KCl, 8 g/L NaCl, 0.2 g/L KH₂PO₄, and 1.15 g/L Na₂HPO₄). The nucleic acid molecule composed of a single nucleic acid strand was, after the dissolving in the D-PBS(-), heated to 90°C. The nucleic acid molecule composed of two or more nucleic acid strands was, after mixing and dissolving a combination of each nucleic acid strands necessary for constituting the nucleic acid molecule in the D-PBS(-),was heated to 90°C. Then, the temperature was gradually decreased for causing annealing between the nucleic acid strands or in the nucleic acid strand to prepare each nucleic acid molecule.

### 2. Preparation of measuring system of the effect of inhibiting the activity

The measuring system of the effect of inhibiting the activity of miR-16 used in the Example is shown in Figure 8. The effect of inhibiting the activity of mir-16 was measured using pDsRed2-mil6-T shown in diagram (a). pDsRed2-mi16-T comprises a gene of fluorescent protein, DsRed2, linked to the downstream of the CMV promoter and three-times repeated sequence completely complementary to miR-16 as a target site of miR-16 (miR-16-T: miR-16-target) is inserted in the 3'-untranslated region of the gene. Accordingly, miR-16-T is also expressed in the expression of the DsRed2 gene in cells as a part of the expression. Here, miR-16-T is an RNAi target of endogenous miR-16. Simultaneously, the translation of DsRed2 is significantly inhibited by RNAi. As a result, only a significantly weak red fluorescence is detected. The fluorescence intensity in this condition was used as a reference value, and the various nucleic acid molecules prepared above were added to this system. If a nucleic acid molecule had the effect of inhibiting the activity of endogenous miR-16, the inhibition of translation of miR-16-T by miR-16 is removed, resulting in the translation of DsRed2, which can be detected as a strong red fluorescence.

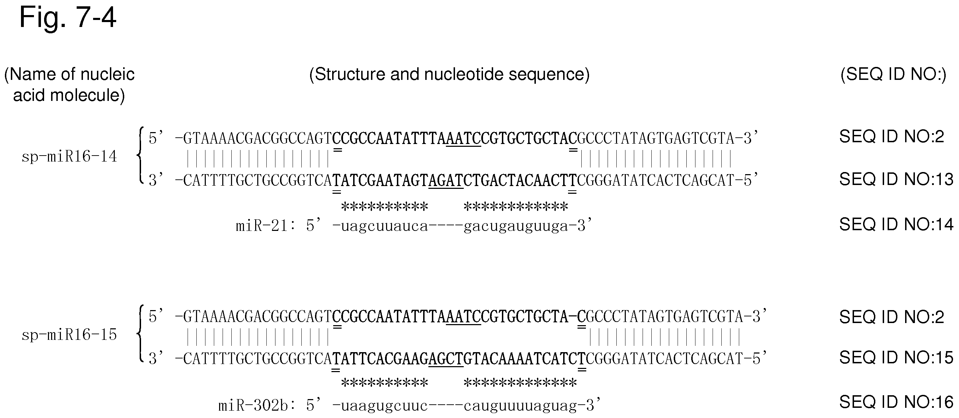

pCAGGS-AFP having the sequence shown in diagram (b) and expressing GFP regardless of the activity of each nucleic acid molecule shown in Figures 7-1 to 7-4 was introduced into HEK293T cells together with the pDsRed2-mi16-T to correct the difference in plasmid transfection efficiency among each sample. That is, when the ratio of the fluorescence intensity of the reference value as the control to the fluorescence intensity of GFP was defined as 1, the normalized DsRed2/GFP ratio (relative value) of each sample was used as the increase in rate of expression.

In preparation of pDsRed2-mil6-T, the multi-cloning site (positions from 1288 to 1363) placed at the 3'-untranslated region (3'-UTR) of a DsRed2 gene in pDsRed2-Cl (Clontech Laboratories, Inc., catalog No. 632407) was modified into 5'-AGATCTCGAGAAGCTTAGATATCGTCGACCCGGGATCCACCGGATCTAGATAACT GA-3' (SEQ ID NO: 42) to produce pDsRed2ERVSMA. This sequence encodes an Arg-Ser-Arg-Glu-Ala-translation stop codon as the C-terminal of DsRed2 protein and comprises EcoRV site (GATATC) adjacent to the stop codon on the downstream side. The pDsRed2-mi16-T was produced by linking a DNA strand, 5'-GTAGCGCCAATATTTACGTGCTGCTACGCCAATATTTACGTGCTGCTACGCCAATA TTTACGTGCTGCTA-3' (SEQ ID NO: 43)/5'-TAGCAGCACGTAAATATTGGCGTAGCAGCACGTAAATATTGGCGTAGCAGCACGT AAATATTGGCGCTAC-3' (SEQ ID NO: 44) or 5'-GTAGCAACGTTGAGGAAGGTGACTGCCAACAACGTTGAGGAAGGTGACTGCCAA CAACGTTGAGGAAGGTGACTGCCAA-3' (SEQ ID NO: 45)/5'-TTGGCAGTCACCTTCCTCAACGTTGTTGGCAGTCACCTTCCTCAACGTTGTTGGCA GTCACCTTCCTCAACGTTGCTAC-3' (SEQ ID NO: 46), prepared by annealing, to the blunt end of pDsRed2ERVSMA cleaved with EcoRV. In pDsRed2-mil6-T, three-times repeated sequence completely complementary to miR-16 (SEQ ID NO: 1) as a target site of miR-16 is inserted. The genetic engineering for the production, for example, extraction and purification of plasmid DNA, preparation of competent cells, transformation of E. coli, DNA cloning, and ligase reaction, was performed in accordance with a procedure known in the art (for example, the method described in Sambrook J., Fritsh E.F., and Maniatis T., (1989), Molecular Cloning: a laboratory manual. Cold Spring Harbor Laboratory, Cold Spring Harbor, Now York).

In order to correct the difference in individual nucleic acid transfection efficiency, pCAGGS-AFP (Momose T. et al., 1999, Dev Growth Differ, 41, 335-44), which expresses GFP regardless of the activity of the double-stranded nucleic acid molecule, was used.

### 3. Transfection method of nucleic acid and fluorescence-measuring method

HEK293T cells were seeded in a 24-well plate at 60000 cells/well and were cultured using DMEM (Wako Pure Chemical Industries, Ltd.) at 37°C in 5% CO₂. After culturing for 24 hours, the cells were transfected with 25 nM of each nucleic acid molecule shown in Figures 7-1 to 7-4, 50 ng of pCAGGS-AFP, and 50 ng of pDsRed2-mil6-T using Lipofectamine™ LTX (Life technologies) in accordance with the protocol of Life technologies. After 48 hours, the cells were destroyed with a buffer TBST (20 mM Tris, pH 7.4, 0.15 M NaCl, 0.05% Triton X-100), followed by centrifugation at 13,000 × G for 30 min. The fluorescence of the supernatant was measured with a fluorescent plate reader (Fluoroskan Ascent FL, Thermofisher Scientific) at excitation and fluorescence wavelengths, respectively, of 485 nm and 538 nm for GFP and 544 nm and 590 nm for DsRed2.

### (Results)

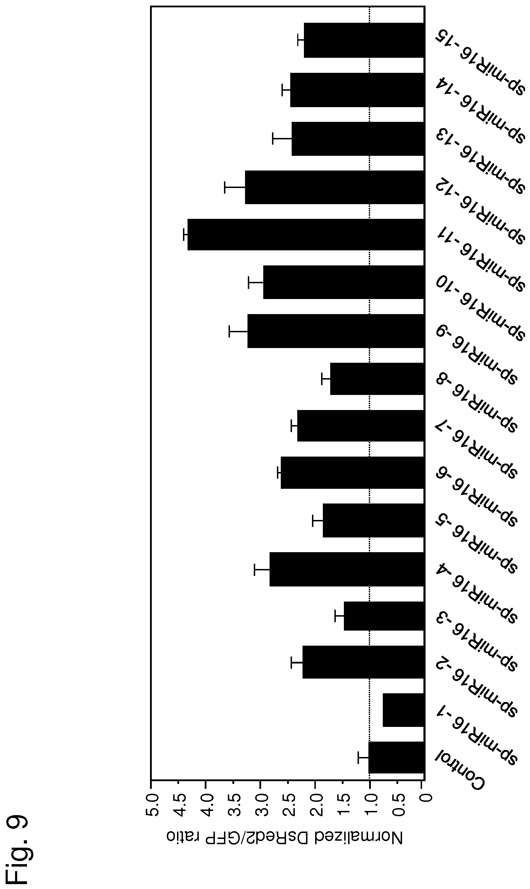

The results are shown in Figure 9. The nucleic acid molecule sp-miR16-1 (here, "sp" means "suppressor", the same shall apply hereinafter) not containing a double-stranded nucleic acid moiety did not show the effect of inhibiting the activity of miR-16. This is not contradicting conventional experimental results that DNA has low avidity to miRNA and therefore shows a low effect of inhibiting the miRNA activity (Meister G., et al., 2004, RNA, 10: 544-550). In contrast, sp-miR16-2 to sp-miR16-16 as the nucleic acid molecules of the present invention all had higher effect of inhibiting the activity of miR-16 compared to the control. The results proved that a nucleic acid molecule comprising a structure, in its molecule, including a single-stranded nucleic acid moiety containing at least one unmodified DNA region consisting of a nucleotide sequence sufficiently complementary to the nucleotide sequence of a functional strand of miR-16 as a target RNAi molecule, and a double-stranded nucleic acid moiety linked to at least one of the 5'-end and the 3'-end of the single-stranded nucleic acid moiety has an action for supressing the activity of miR-16. It has been revealed that in particular, a nucleic acid molecule in which a double-stranded nucleic acid moiety is linked to the 3'-end of a single-stranded nucleic acid moiety has a relatively high effect of inhibiting the activity of miR-16 compared to that of a nucleic acid molecule in which the double-stranded nucleic acid moiety is linked to the 5'-end of the single-stranded nucleic acid moiety.

### <Example 2: Preparation of the nucleic acid molecule for inhibiting the activity of miR-16 and verification of its effect of inhibiting the activity (2)>

### (Purpose)

Whether the effect of inhibiting the activity of miR-16 by the nucleic acid molecule for inhibiting the activity of miR-16 of the present invention observed in Example 1 is concentration-dependent or not and whether the effect is specific to the nucleic acid molecule for inhibiting the activity of miR-16 or not were verified.

### (Method)

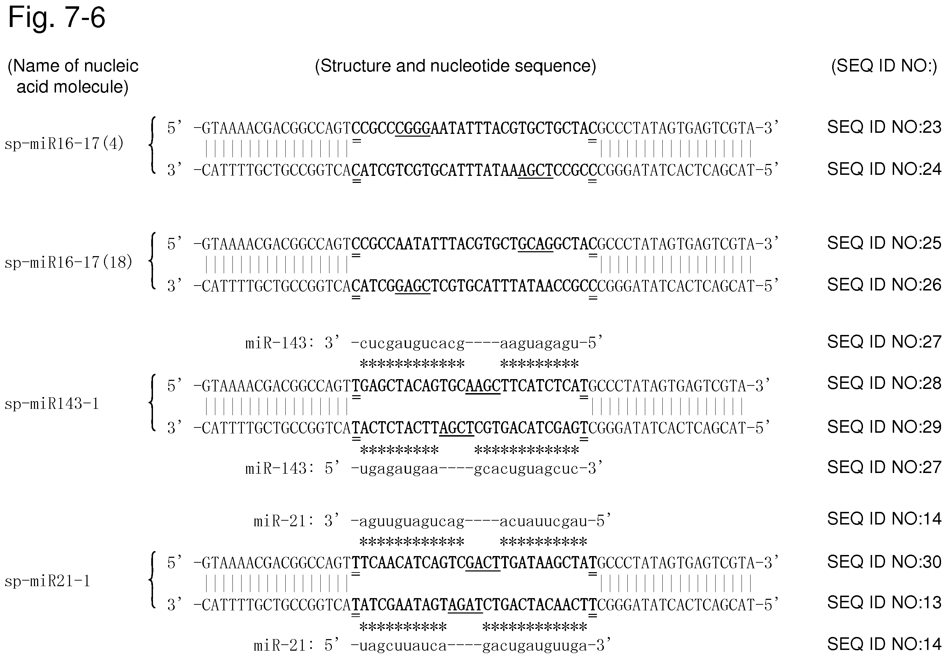

The basic method was performed in accordance with Example 1. As the nucleic acid molecule for inhibiting the activity of miR-16, sp-miR16-11 used in Example 1 was used. As controls, sp-miR16-1 not having a double-stranded nucleic acid moiety and sp-miR143-1, a nucleic acid molecule for inhibiting the activity of miR-143, were used. The sp-miR143-1 was prepared based on the nucleotide sequence shown in Figure 7-6 by the same method as in Example 1. In addition, the preparation of the measuring system of the effect of inhibiting the activity, transfection method of nucleic acid, and the fluorescence-measuring method were performed in accordance with those in Example 1 except that the sp-miR16-11 and sp-miR143-1 were added to the cultured cells at a concentration of 5, 10, or 25 nM and that the sp-miR143-1 was added to the cultured cells at a concentration of 25 or 50 nM.

### (Results)

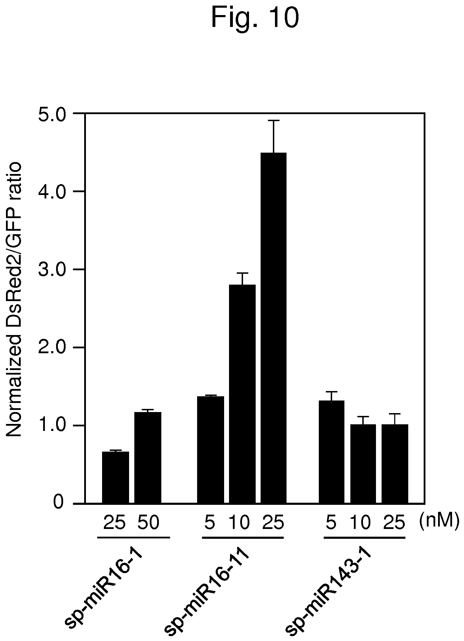

The results are shown in Figure 10. In sp-miR16-1 not containing a double-stranded nucleic acid moiety, the relative value at a concentration of 25 nM was not higher than those of the controls, and even at a concentration of 50 nM, the effect of inhibiting the activity of miR-16 was hardly observed. This suggests that at a low concentration of 25 nM, a single-stranded nucleic acid moiety only is almost impossible to bind to miR-16. In contrast, sp-miR16-11 as a nucleic acid molecule for inhibiting the activity of miR-16 showed the effect of inhibiting the activity of miR-16 compared to the control at a very small amount of 5 nM, and it was proved that the strength increases depended on the concentration of the nucleic acid molecule. In sp-miR143-1, the effect of inhibiting the activity of miR-16 was not observed at every concentration. These results revealed that the nucleic acid molecule for inhibiting the activity of miR-16 shown in Example 1 has the effect of inhibiting the activity of miR-16 even at a low concentration; that the strength of the effect concentration-dependently increases; and that the effect of inhibiting the activity of miR-16 is specific to the nucleic acid molecule for inhibiting the activity of miR-16 and is not observed in other nucleic acid molecule for inhibiting the activity of miRNA, such as the nucleic acid molecule for inhibiting the activity of miR-143.

### <Example 3: Verification of the effect of inhibiting the activity by cultured cells of the effect of inhibiting the activity of miR-16>

### (Purpose)

Whether the effect of inhibiting the activity of miR-16 of the nucleic acid molecule of the present invention depends on the cells into which the molecule is introduced or not was verified.

### (Method)

As the nucleic acid molecules, sp-miR16-11 described in Example 1 and sp-miR143-1 described in Example 2 were used. The cultured cells used were HEK293T cells, HepG2 cells, and MCF7 cells, which all had miR-16 as endogenous miRNA. The culturing conditions of each cultured cell, the amounts and transfection method of the nucleic acid molecules, and the fluorescence-measuring method were performed in accordance with Example 1.

### (Results)

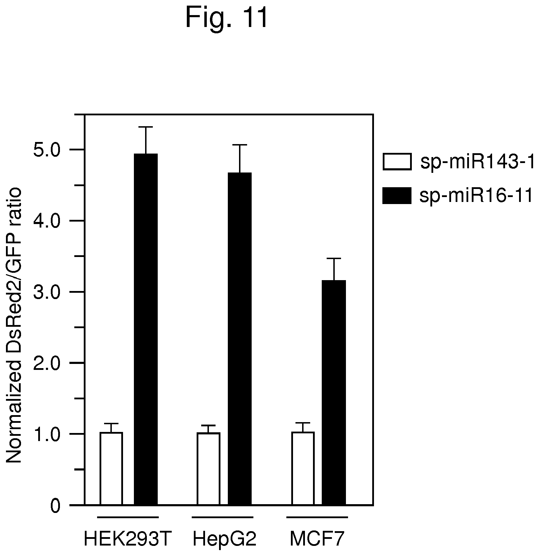

The results are shown in Figure 11. As shown in this graph, it was demonstrated that sp-miR16-11 as the nucleic acid molecule of the present invention had a high effect of inhibiting the activity of miR-16 in all cultured cells having endogenous miR-16, regardless of the type of the cells. In contrast, no effect of inhibiting the activity of miR-16 was observed in sp-miR143-1 as the nucleic acid molecule for inhibiting the activity of miR-143 in all cultured cells. Thus, it was proved that the effect is specific to the nucleic acid molecule for inhibiting the activity of miR-16.

### <Example 4: Nucleotide length of mismatch site in the nucleic acid molecule for inhibiting the activity of RNAi and the effect of inhibiting the activity>

### (Purpose)

In the nucleic acid molecule for inhibiting the activity of an RNAi molecule of the present invention each having a mismatch site in the unmodified DNA region, the effect of inhibiting the activity of an RNAi molecule from the nucleotide length (the number of nucleotide residues) of the mismatch site was verified.

### (Method)

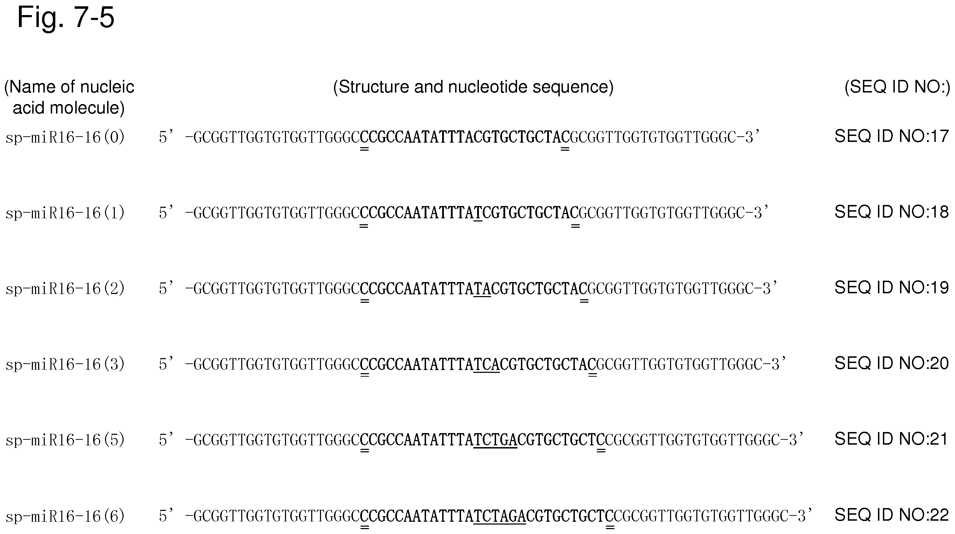

As the nucleic acid molecules, sp-miR16-16(0), sp-miR16-16(1), sp-miR16-16(2), sp-miR16-16(3), sp-miR16-16(4), sp-miR16-16(5), and sp-miR16-16(6) shown in Figure 7-5 were used. The number in parentheses of the name of each nucleic acid molecule means the number of nucleotides of the mismatch site. The mismatch site was placed between A at the 12th position and C at the 13th position from the 5'-end of an unmodified DNA region not having a mismatch site, i.e., the unmodified DNA region of sp-miR16-16(0), which is completely the same as a functional strand of miR-16. The nucleic acid molecules were each prepared based on the nucleotide sequences shown in Figure 7-5 by the same method as in Example 1. The cultured cells used were HEK293T cells. The culturing conditions of each cultured cell, the amounts and transfection method of the nucleic acid molecules, and the fluorescence-measuring method were performed in accordance with Example 1.

### (Results)

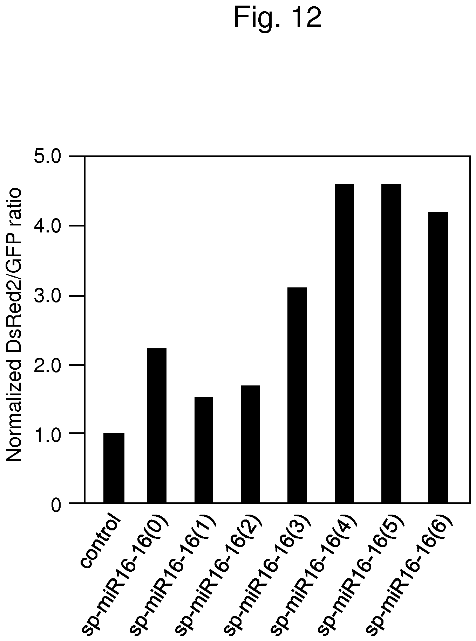

The results are shown in Figure 12. As shown in this graph, it was revealed that the nucleic acid molecules of the present invention had the effect of inhibiting the activity of miR-16, regardless of the presence of a mismatch site and the nucleotide length of the mismatch site.

### <Example 5: Position of mismatch site in the nucleic acid molecule for inhibiting the activity of an RNAi molecule and the effect of inhibiting the activity>

### (Purpose)

In the nucleic acid molecule for inhibiting the activity of an RNAi molecule of the present invention each having a mismatch site in the unmodified DNA region, the position of the mismatch site and the effect of inhibiting the activity of RNAi molecule was verified.

### (Method)

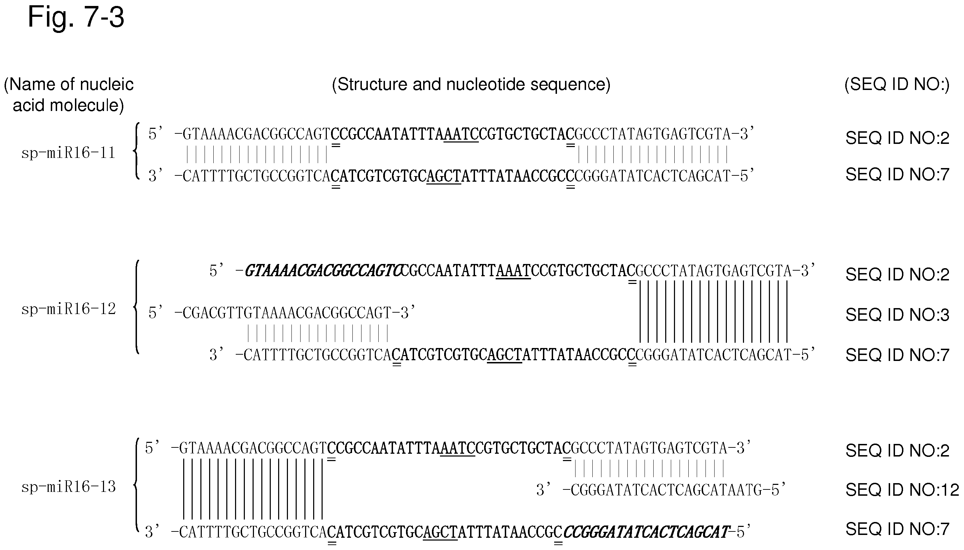

As the nucleic acid molecules, sp-miR16-11 shown in Figure 7-3 and sp-miR16-17(4) and sp-miR16-17(18) shown in Figure 7-6 were used. The numbers in parentheses of sp-miR16-17(4) and sp-miR16-17(18) show the positions of the mismatch sites. For example, in sp-miR16-17(4), a mismatch site was placed between the nucleotides at the 4th and 5th positions from the 5'-end of the unmodified DNA region, in the nucleic acid molecule completely the same as that of a functional strand of miR-16. In this Example, sp-miR16-11 corresponds to sp-miR16-17(12). The nucleic acid molecules were each produced based on the nucleotide sequences shown in Figures 7-3 and 7-6 by the same method as in Example 1. The cultured cells used were HEK293T cells. The culturing conditions of each cultured cell, the amounts and transfection method of the nucleic acid molecules, and the fluorescence-measuring method were performed in accordance with Example 1.

### (Results)

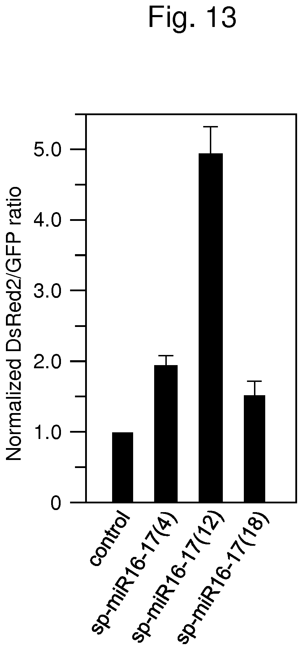

The results are shown in Figure 13. As shown in this graph, it was demonstrated that the nucleic acid molecules of the present invention all had the effect of inhibiting the activity of miR-16, regardless of the position of the mismatch site. It was also revealed that the effect of inhibiting the activity was high when the mismatch site was placed in the central region (the 9th to 14th positions) of the unmodified DNA region.

### <Example 6: Nucleotide length of linking region in the nucleic acid molecule for inhibiting the activity of an RNAi molecule and the effect of inhibiting the activity>

### (Purpose)

In the case of the nucleic acid molecule for inhibiting the activity of an RNAi molecule of the present invention having a linking region in the single-stranded nucleic acid moiety, the nucleotide length of the linking region and the effect of inhibiting the activity of an RNAi molecule was verified.

### (Method)

As the nucleic acid molecules, sp-miR16-18(0), sp-miR16-18(1), sp-miR16-18(2), sp-miR16-18(3), sp-miR16-18(4), and sp-miR16-18(5) shown in Figure 7-7 were used. The number in parentheses means the number of nucleotides of the linking region. The nucleotides of the linking regions were all T, except that the nucleotide of sp-miR16-18(1) was C. In all of the nucleic acid molecule for inhibiting the activity of miR-16 used in this Example as the nucleic acid molecule for inhibiting the activity of an RNAi molecule , the double-stranded nucleic acid moieties were each composed of a single strand; the nucleotide sequence thereof includes a sequence, 5'-GGTTGGTGTGGTTGG-3' (SEQ ID NO: 41), that can form a G-quartet; and the unmodified DNA regions each have a mismatch site, 5'-TCGA-3', having a length of four nucleotides at the same position. The nucleic acid molecules were prepared based on the nucleotide sequences shown in Figure 7-7 by the same method as in Example 1. The cultured cells used were HEK293T cells. The culturing conditions of each cultured cell, the amounts and transfection method of the nucleic acid molecules, and the fluorescence-measuring method were performed in accordance with Example 1.

### (Results)

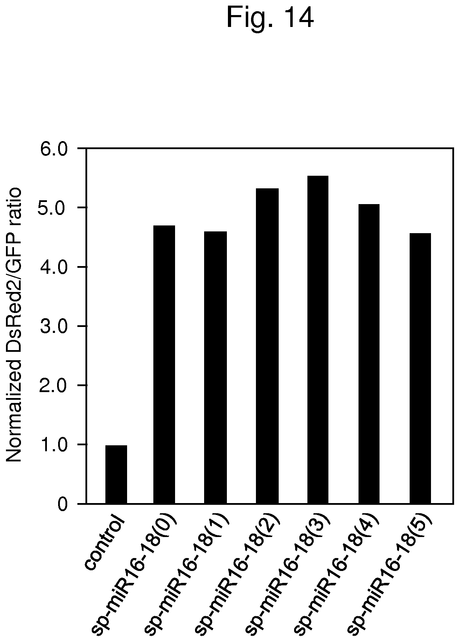

The results are shown in Figure 14. As shown in this graph, it was demonstrated that the nucleic acid molecules of the present invention all had approximately the same degrees of the effect of inhibiting the activity of miR-16, regardless of the nucleotide length of the linking region.

### <Example 7: Preparation of the nucleic acid molecule for inhibiting the activity of miR-143 and verification of its effect of inhibiting the activity>

### (Purpose)

Whereas in Examples 1 to 6, the effect of inhibiting the activity of the nucleic acid molecule of which the target RNAi molecule was miR-16 were verified, whether the nucleic acid molecule for inhibiting the activity of an RNAi molecule having a constitution of the present invention has a similar effect of specifically inhibiting the activity of a target RNAi molecule, even if miRNA other than miR-16 is used as a target RNAi molecule, was verified.

### (Method)

Nucleic acid molecules (sp-miR143-1 and sp-miR21-1), of which target RNAi molecules were respectively miR-143 and miR-21, shown in Figure 7-6 were prepared by the same method as in Example 1 and were introduced into HEK293T cells. The basic method of the effect of inhibiting the activity was performed in accordance with Example 1, except that 25 nM of miR-143 was mixed with the nucleic acid molecule described above and was introduced into the cells together with the nucleic acid molecule, since HEK293T cells hardly express the endogenous miR-143.

### (Results)

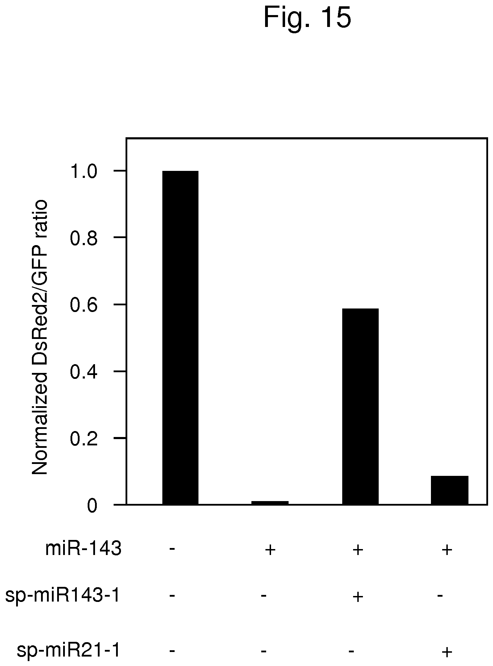

The results are shown in Figure 15. As shown in the graph, the expression of pDsRed2 was significantly suppressed by adding the exogenous miR-143. However, the activity of miR-143 was suppressed by simultaneously adding sp-miR143-1, and the expression of pDsRed2 was recovered to about 60% of that when the exogenous miR-143 was not added. In contrast, the expression of pDsRed2 was recovered to only about 10% when sp-miR21-1 was simultaneously added. This result proved that the nucleic acid molecule for inhibiting the activity of an RNAi molecule of the present invention can specifically inhibit the activity of a target RNAi molecule, regardless of the type of the target RNAi molecule.

### <Example 8: Analysis of avidity between the nucleic acid molecule for inhibiting the activity of an RNAi molecule of the present invention and miRNA>

### (Purpose)

The avidity between the nucleic acid molecule for inhibiting the activity of an RNAi molecule of the present invention and miRNA was analyzed by surface plasmon resonance (SPR) measuring method.

### (Method)

### 1. Preparation of nucleic acid molecule

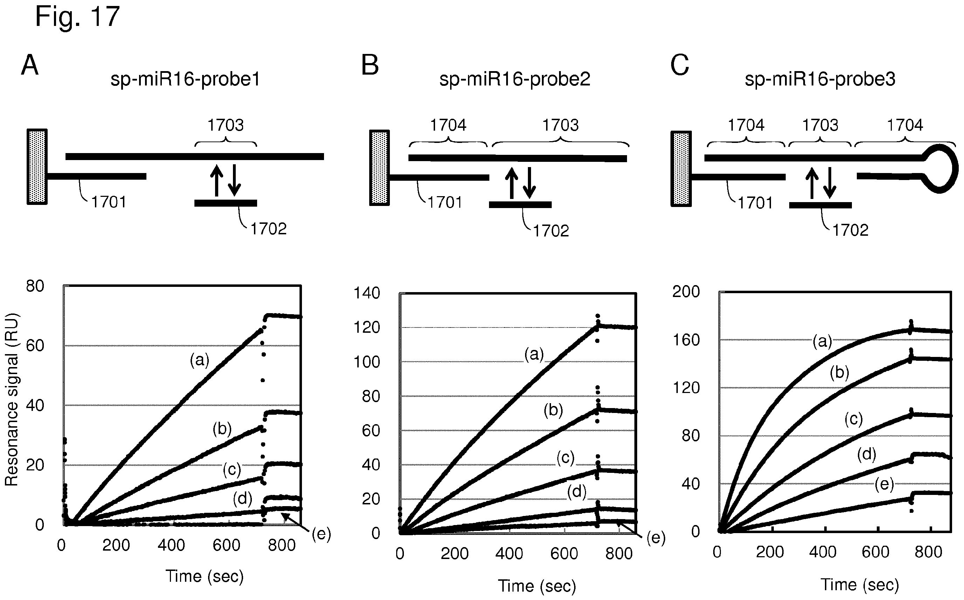

Figure 17 shows the names, structures, and nucleotide sequences of the nucleic acid molecules used in the Example. The nucleic acid strands (sp-miR16-probe1, sp-miR16-probe2, and sp-miR16-probe3) consisting of DNA oligonucleotides constituting each nucleic acid molecule and the anchor nucleic acid strands of which the 5'-ends were biotinylated were chemically synthesized by FASMAC Co., Ltd. under contract. Here, sp-miR16-probe1 is a nucleic acid molecule composed of a single-stranded nucleic acid moiety only and not having a double-stranded nucleic acid moiety; sp-miR16-probe2 is a nucleic acid molecule of the present invention comprising a single-stranded nucleic acid moiety and a double-stranded nucleic acid moiety composed of two nucleic acid strands at the 3'-end of the single-stranded nucleic acid moiety; and sp-miR16-probe3 is a nucleic acid molecule of the present invention comprising a single-stranded nucleic acid moiety and a double-stranded nucleic acid moiety composed of two nucleic acid strands and a double-stranded nucleic acid moiety composed of a single nucleic acid strand having a loop region at the 5'-end and the 3'-end, respectively, of the single-stranded nucleic acid moiety. These nucleic acid molecules, excluding the anchor nucleic acid strands, were not modified. The synthesized oligonucleotides were each dissolved in D-PBS(-) (0.2 g/L KCl, 8 g/L NaCl, 0.2 g/L KH₂PO₄, and 1.15 g/L Na₂HPO₄). After the dissolving, sp-miR16-probe3 was heated to 90°C and was gradually cooled to cause annealing of the 5'-end part in the nucleic acid strand to form a double-stranded nucleic acid moiety having a loop region.

### 2. Preparation of sensor chip for SPR measurement

First, streptavidin was immobilized to Biacore 3000 sensor chip CM5 (GE Healthcare) with carbodiimide coupling, which was specifically performed in accordance with the standard protocol of GE Healthcare. Next, the anchor nucleic acid strand was added thereto to immobilize the anchor nucleic acid strand onto the sensor chip with biotin-streptavidin binding. Subsequently, the prepared sp-miR16-probe1, sp-miR16-probe2, or sp-miR16-probe3 was introduced. These nucleic acid molecules each have a sequence complementary to the anchor nucleic acid strand at the 3'-end part and therefore are each immobilized onto the sensor chip thorough base pairing with the anchor nucleic acid strand.

### 3. Measurement of affinity with Biacore 3000

SPR measurement was performed with Biacore 3000 (GE Healthcare). mir-16 which was dissolved at 3.125 nM, 6.25 nM, 12.5 nM, 25 nM, or 50 nM was injected and binding amount was measured, respectively. The binding constant was calculated in accordance with the standard protocol of GE Healthcare.

### (Results)

The results are shown in Figure 17 and Table 1.

**[Table 1]**

| probe | *kₐ*(M⁻¹s⁻¹) | *k_{d}*(s⁻¹) | K_{D}(µM) |
|---|---|---|---|
| sp-miR 16-probe1 | 1.9 × 10² | 1.9 × 10⁻⁴ | 1.0 |
| sp-miR 16-probe2 | 1.4 × 10⁴ | 9.6 × 10⁻⁵ | 7.1 × 10⁻³ |
| sp-miR16-probe3 | 9.2 × 10⁴ | 5.0 × 10⁻⁵ | 1.9 × 10⁻⁴ |

In Figure 17, graphs A to C show binding affinities of sp-miR16-probe1, sp-miR16-probe2, and sp-miR16-probe3 to miR-16, respectively. In addition, table 1 shows the binding properties of each nucleic acid molecule obtained from the results shown in Figure 17.

As shown in Table 1, in sp-miR16-probel consisting of only a single-stranded nucleic acid moiety composed of DNA, the K_{D} value was 1 µM. On the other hand, in sp-miR16-probe2 having a double-stranded nucleic acid moiety at one end of a single-stranded nucleic acid moiety and sp-miR16-probe3 having double-stranded nucleic acid moieties at both ends of a single-stranded nucleic acid moiety, the K_{D} values were 7.1 nM and 0.54 nM, respectively. This proved that although nucleic acid composed of a single-stranded DNA only had low avidity to miRNA as conventionally recognized in the art, nucleic acid molecules having structures of the present invention had high avidity to miRNA such that the K_{D} values were increased by three or more orders of magnitude. This suggests that the molecule can bind to miRNA even if the concentration is significantly low. In addition, the effect of inhibiting the activity of miRNA of the nucleic acid molecule of the present invention has been already revealed in Examples above.

The results described above demonstrate that the present invention can inexpensively provide the nucleic acid molecule for inhibiting the activity of a target RNAi molecule, the nucleic acid molecule being composed of DNA which is a natural nucleic acid being highly stable *in vivo,* capable of being synthesized in a large quantity, and having high safety *in vivo* and being capable of binding to miRNA even at a low concentration to inhibit the activity.

### <Example 9: Comparison of the effect of inhibiting the activity of the nucleic acid molecule for inhibiting the activity of miR-16 and modified nucleic acid molecule>

### (Purpose)

The effects of inhibiting the activity of endogenous miR-16 were compared between the nucleic acid molecule for inhibiting the activity of miR-16 having a structure of the present invention and a miR-16 inhibitor comprising a bridged nucleic acid (BNA or LNA) as non-natural nucleic acid or RNA chemically modified with 2'-OMe.

### (Method)

The structures and nucleotide sequences of nucleic acid molecules used in this Example are shown in Figure 18. The nucleic acid molecule for inhibiting the activity of miR-16 (sp-miR16-19) was composed of nucleic acid molecules set forth in SEQ ID NOs: 47 and 48. A miR-16 inhibitor (BNA-miR16-sup) containing BNA was consisting of a nucleic acid molecule containing BNA set forth in SEQ ID NO: 49 (synthesized by Japan Bio Services Co., Ltd. under contract). Further, A miR-16 inhibitor (OMe-miR16-sup) consisting of RNA chemically modified with 2'-OMe is a nucleic acid molecule set forth in SEQ ID NO: 50 (synthesized by Japan Bio Services Co., Ltd. under contract). As a control, a nucleic acid molecule for inhibiting the activity of miR-302cd (sp-miR302cd) of which the target is miR302cd and the nucleic acid molecules set forth in SEQ ID NOs: 51 and 52 was used.

HEK293T cells (12000 cells) were seeded in a 96-well plate. On the following day, the HEK293T cells were transfected with 15 ng of pDsRed2-mi16-T constructed in Example 1, 5 ng of pCAGGS-AFP as a GFP expression vector, for determining the transfection efficiency and correcting the effect of inhibiting the activity of miR-16 , and 10 nM or 25 nM of sp-miR16-19, BNA-miR16-sup, OMe-miR16-sup, or sp-miR302cd using Lipofectamine™ LTX (Life technologies). After further two days, the cells were pulverized and extracted with an RIPA buffer (25 mM Tris-HCl, pH 7.6, 150 mM NaCl, 1% NP-40, 1% sodium deoxycholate, 0.1% SDS), followed by centrifugation. The supernatant was collected, and the fluorescence intensity was measured.

### (Results)

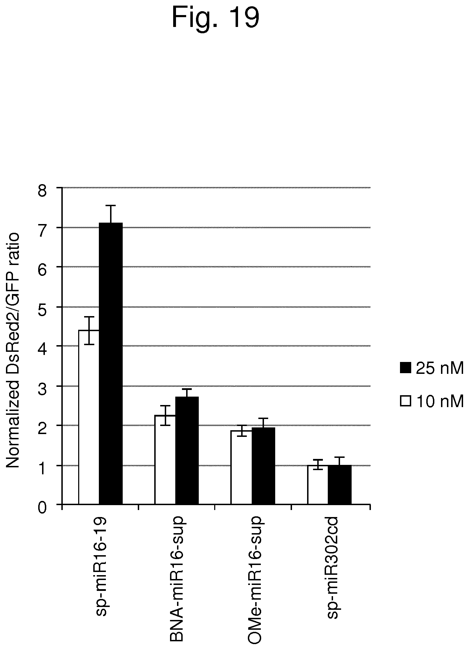

The results are shown in Figure 19. The effect of sp-miR16-19 for inhibiting the miR-16 activity was two times higher than those of conventional miRNA inhibitors (BNA-miR16-sup and OMe-miR16-sup) containing BNA or RNA chemically modified with 2'-OMe. The results revealed that the effect increases concentration-dependently as in the results of Example 2.

### <Example 10: Effect of suppressing proliferation of cancer cells by the nucleic acid molecule for inhibiting the activity of miR-21 (1)>

### (Purpose)

The effect of suppressing the proliferation of cancer cells by the nucleic acid molecule for inhibiting the activity of miR-21 having the structure of the present invention was verified. It is known that miR-21 is highly expressed as Onco-miRNA (oncomir) in various cancer cells (Cho W.C., 2007, Mol Cancer, 6, 60). Since miR-21 has an activity of suppressing apoptosis, cancer cells highly expressing miR-21 can avoid apoptosis by its activity. Accordingly, whether the activity of miR-21 can be suppressed by introducing the nucleic acid molecule for inhibiting the activity of miR-21 into cancer cells and thereby the apoptosis action is recovered to suppress the proliferation of the cancer cells was investigated.

### (Method)

As the cancer cells, human breast cancer-derived cell line MCF7 was used. In addition, as the nucleic acid molecule for inhibiting the activity of miR-21, sp-miR21-1 shown in Figure 7-6 was used.

MCF7 cells (18000 cells) were seeded in a 96-well plate. On the following day, the MCF7 cells were transfected with 5 ng of pCAGGS-AFP and 25 nM or 0 nM of sp-miR21-1 using Lipofectamine™ LTX (life technologies). The cells 1, 2, 3, and 5 days after the transfection were subjected to WST assay using Cell Counting Kit-8 (Dojindo Laboratories), and absorbance at 450 nm was measured. During the experiment, the culture medium was replaced with a fresh medium on the first and the third day after the transfection.

### (Results)

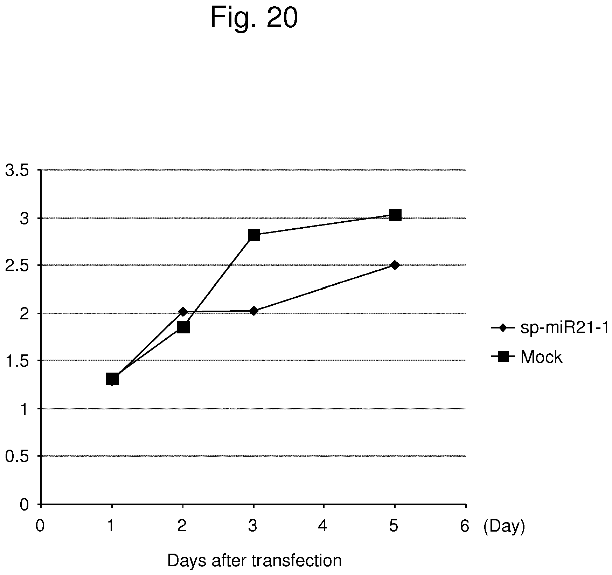

The results are shown in Figure 20. As shown in this graph, it was revealed that in the MCF7 cells (Mock) to which sp-miR21-1 was introduced, the cell proliferation was suppressed on from the 2nd day after the transfection, whereas in MCF7 cells not containing sp-miR21-1, the absorbance at 450 nm in the WST assay, i.e., the number of cells, increased with the day of culturing. This result demonstrates that the nucleic acid molecule for inhibiting the activity of an RNAi molecule of the present invention inhibites the activity of a target RNAi molecule involved in cancer *in vivo* and thereby can effectively suppress the proliferation of cancer cells.

### <Example 11: Effect of suppressing proliferation of cancer cells by the nucleic acid molecule for inhibiting the activity of miR-21 (2)>

### (Purpose)

It was verified that the nucleic acid molecule for inhibiting the activity of miR-21 having a structure of the present invention has a similar effect *in vivo* as that in Example 10, regardless of the structure (secondary structure and/or tertiary structure).

### (Method)

The basic method was performed in accordance with Example 10. As the nucleic acid molecule for inhibiting the activity of miR-21, sp-miR21-YMB (SEQ ID NO: 53) shown in Figure 18 was used. In sp-miR21-YMB, the double-stranded nucleic acid moieties placed at the 5'-end and the 3'-end of a single-stranded nucleic acid moiety given in bold letters in Figure 18 each form a hairpin-shaped stem-and-loop structure by intramolecular folding.

### (Results)

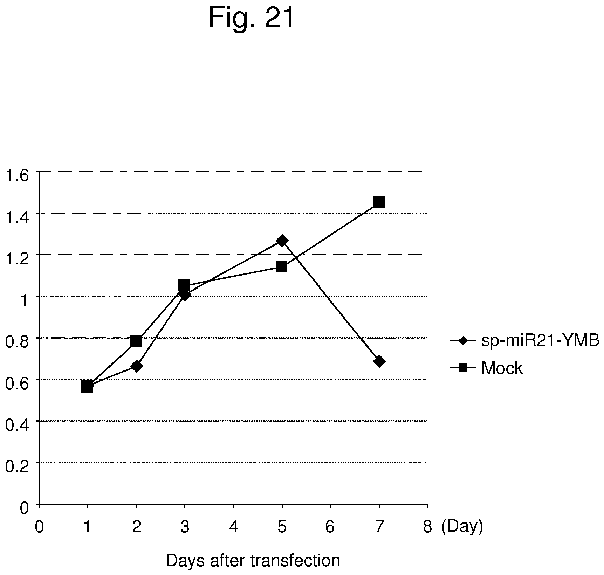

As shown in Figure 21, the same tendency as that shown in Figure 20 was observed. However, whereas sp-miR21-1 showed the effect of suppressing cell proliferation on the 2nd day after the introduction into MCF7 cells, sp-miR21-YMB hardly showed any difference from the control (Mock) by the 5th day after the introduction into MCF7 cells. However, after that, a remarkable effect of suppressing cell proliferation higher than that of sp-miR21-1 was observed. The time lag until the appearance of the effect has not been clarified yet, but it was proved that the nucleic acid molecule for inhibiting the activity of an RNAi molecule of the present invention can inhibit the activity of the same target RNAi molecule *in vivo,* regardless of the secondary structure or tertiary structure.

### <Example 12: Effect of inhibiting the activity of exogenous miR-125b>

### (Purpose)

Many of Examples above, excluding miR-143 of Example 7, verified the activities of the nucleic acid molecule for inhibiting the activity of an RNAi molecule of the present invention inhibiting the activities of RNAi molecules (miRNA) resident in cells only as a target.

However, RNAi molecules such as miRNA, siRNA, and shRNA may be exogenously introduced into cells as nucleic acid medicine. Accordingly, in this Example, whether the nucleic acid molecule for inhibiting the activity of an RNAi molecule of the present invention can also inhibit the activity of an exogenous RNAi molecule as a target molecule was verified.

### (Method)

As the exogenous RNAi molecule, miR-125b was used. The structures and the nucleotide sequences of the nucleic acid molecule for inhibiting the activity of an RNAi molecule used in this Example are shown in Figure 18. The nucleic acid molecule for inhibiting the activity of miR-125b (sp-miR125b-YMB) is composed of the nucleic acid molecule set forth in SEQ ID NO: 54. In this nucleic acid molecule, as in sp-miR21-YMB, the double-stranded nucleic acid moieties placed at the 5'-end and the 3'-end of a single-stranded nucleic acid moiety given in bold letters each form a hairpin-shaped stem-and-loop structure by intramolecular folding. As a control of the nucleic acid molecule for inhibiting the activity of an RNAi molecule, a nucleic acid molecule for inhibiting the activity of miR-39 (sp-miR39-YMB) composed of the nucleic acid molecule set forth in SEQ ID NO: 55 inhibiting the activity of target miR-39 was used.

HEK293T cells (12000 cells) were seeded in a 96-well plate. On the following say, the HEK293T cells were transfected with 15 ng of DSRed expression plasmid, pDsRed2-mi125b-T, containing a target of miR-125b, 5 ng of a GFP expression vector, pCAGGS-AFP, for determining the transfection efficiency and correcting the effect of inhibiting the activity of mir-125b, 15 ng of sp-miR125b-YMB or sp-miR39-YMB, and 5 ng of miR-125b as a target RNAi molecule, using Lipofectamine™ LTX (Life technologies). After further two days, the cells were pulverized and extracted with an RIPA buffer, followed by centrifugation. The supernatant was collected, and the fluorescence intensity was measured (n = 3). The construction of pDsRed2-mi125b-T was performed in accordance with pDsRed2-mi16-T.

### (Results)

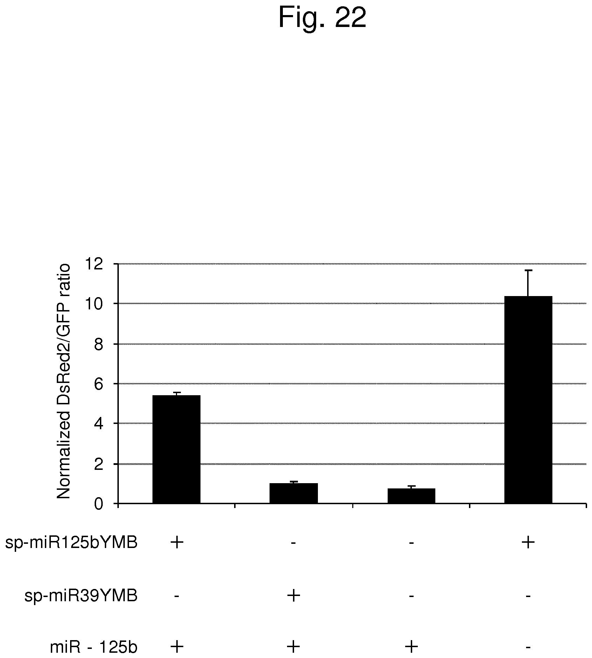

The results are shown in Figure 22. It was revealed that even in the case of RNAi molecule exogenously introduced into cells, the nucleic acid molecule for inhibiting the activity of an RNAi molecule of the present invention can specifically inhibit the activity of a target RNAi molecule.

[DELETED]

### Reference Signs List

- 101, 201, 1703:: single-stranded nucleic acid moiety
- 102, 202, 1704:: double-stranded nucleic acid moiety
- 103, 203:: unmodified DNA region
- 204:: linking region
- 205:: flanking region
- 206:: mismatch site
- 207:: spacer region
- 1701:: anchor nucleic acid strand
- 1702:: miRNA (miR-16)

## Claims

1. A nucleic acid molecule for inhibiting an activity of a target RNAi molecule, comprising:
a single-stranded nucleic acid moiety including one or more unmodified DNA regions consisting of a nucleotide sequence sufficiently complementary to a nucleotide sequence of a functional strand having the activity in the target RNAi molecule; and
a double-stranded nucleic acid moiety linked to at least one of the 5'-end and the 3'-end of the single-stranded nucleic acid moiety,
wherein at least one unmodified DNA region comprises a mismatch site of 3 to 8 successive nucleotides that forms a loop when the unmodified DNA region is allowed to form base pairs with the functional strand.

2. The nucleic acid molecule according to Claim 1, wherein at least one unmodified DNA region has a length of 18 to 35 nucleotides.

3. The nucleic acid molecule according to Claim 1 or 2, wherein the single-stranded nucleic acid moiety comprises two or more same or different unmodified DNA regions.

4. The nucleic acid molecule according to Claim 3, further comprising a spacer region consisting of nucleic acid having a length of 1 to 10 nucleotides between two of the unmodified DNA regions and linking the regions together.

5. The nucleic acid molecule according to any one of Claims 1 to 4, comprising two or more same or different single-stranded nucleic acid moieties.

6. The nucleic acid molecule according to any one of Claims 1 to 5, wherein the single-stranded nucleic acid moiety or moieties comprise a linking region consisting of nucleic acid having a length of 1 to 10 nucleotides for mediating the linkage between one of the unmodified DNA regions and the double-stranded nucleic acid moiety.

7. The nucleic acid molecule according to any one of Claims 1 to 6, wherein the RNAi molecule is siRNA, shRNA, or miRNA.

8. The nucleic acid molecule according to any one of Claims 1 to 7, wherein the double-stranded nucleic acid moiety has a length of 5 to 25 nucleotides.

9. The nucleic acid molecule according to any one of Claims 1 to 8, wherein the double-stranded nucleic acid moiety has a mismatch site of one nucleotide or 2 to 6 successive nucleotides.

10. The nucleic acid molecule according to any one of Claims 1 to 9, wherein the double-stranded nucleic acid moiety has a loop region consisting of nucleic acid having a length of 3 to 10 nucleotides and linking the 3'-end of one nucleic acid strand of the double-stranded nucleic acid moiety and the 5'-end of the other nucleic acid strand.

11. The nucleic acid molecule according to any one of Claims 1 to 10, wherein the double-stranded nucleic acid moiety has a nick in one or both of the nucleic acid strands.

12. The nucleic acid molecule according to any one of Claims 1 to 11, wherein the double-stranded nucleic acid moiety has a triplex or a quadruplex.

13. The nucleic acid molecule according to any one of Claims 1 to 12, being composed of DNA only.

14. A pharmaceutical composition comprising a nucleic acid molecule according to any one of Claims 1 to 13 as an active ingredient.

## Patentansprüche

1. Nucleinsäuremolekül zur Hemmung einer Aktivität eines RNAi-Zielmoleküls, umfassend:
eine einzelsträngige Nucleinsäuregruppierung, die eine oder mehrere nicht modifizierte DNA-Regionen umfasst, die aus einer Nucleotidsequenz bestehen, die ausreichend komplementär zu einer Nucleotidsequenz eines funktionalen Strangs ist, der die Aktivität im RNAi-Zielmolekül aufweist; und
eine doppelsträngige Nucleinsäuregruppierung, die an zumindest eines aus dem 5'-Ende und dem 3'-Ende der einzelsträngigen Nucleinsäuregruppierung gebunden ist,
wobei zumindest eine nicht modifizierte DNA-Region eine Fehlpaarungsstelle aus 3 bis 8 aufeinanderfolgenden Nucleotiden umfasst, die eine Schleife bildet, wenn es möglich ist, dass die nicht modifizierte DNA-Region Basenpaare mit dem funktionalen Strang bildet.

2. Nucleinsäuremolekül nach Anspruch 1, wobei zumindest eine nicht modifizierte DNA-Region eine Länge von 18 bis 35 Nucleotiden aufweist.

3. Nucleinsäuremolekül nach Anspruch 1 oder 2, wobei die einzelsträngige Nucleinsäuregruppierung zwei oder mehr gleiche oder unterschiedliche nicht modifizierte DNA-Regionen umfasst.

4. Nucleinsäuremolekül nach Anspruch 3, das des Weiteren eine Spacerregion umfasst, die aus einer Nucleinsäure mit einer Länge von 1 bis 10 Nucleotiden besteht, die sich zwischen zwei der nicht modifizierten DNA-Regionen befindet und die Regionen miteinander verbindet.

5. Nucleinsäuremolekül nach einem der Ansprüche 1 bis 4, das zwei oder mehr gleiche oder unterschiedliche einzelsträngige Nucleinsäuregruppierungen umfasst.

6. Nucleinsäuremolekül nach einem der Ansprüche 1 bis 5, wobei die einzelsträngige(n) Nucleinsäuregruppierung oder -gruppierungen eine Bindungsregion umfasst/umfassen, die aus einer Nucleinsäure mit einer Länge von 1 bis 10 Nucleotiden besteht, um die Bindung zwischen einer der nicht modifizierten DNA-Regionen und der doppelsträngigen Nucleinsäuregruppierung zu vermitteln.

7. Nucleinsäuremolekül nach einem der Ansprüche 1 bis 6, wobei das RNAi-Molekül siRNA, shRNA oder miRNA ist.

8. Nucleinsäuremolekül nach einem der Ansprüche 1 bis 7, wobei die doppelsträngige Nucleinsäuregruppierung eine Länge von 5 bis 25 Nucleotiden aufweist.

9. Nucleinsäuremolekül nach einem der Ansprüche 1 bis 8, wobei die doppelsträngige Nucleinsäuregruppierung eine Fehlpaarungsstelle aus einem Nucleotid oder 2 bis 6 aufeinanderfolgenden Nucleotiden aufweist.

10. Nucleinsäuremolekül nach einem der Ansprüche 1 bis 9, wobei die doppelsträngige Nucleinsäuregruppierung eine Schleifenregion aufweist, die aus einer Nucleinsäure mit einer Länge von 3 bis 10 Nucleotiden besteht und das 3'-Ende eines Nucleinsäurestrangs der doppelsträngigen Nucleinsäuregruppierung und das 5'-Ende des anderen Nucleinsäurestrangs miteinander verbindet.

11. Nucleinsäuremolekül nach einem der Ansprüche 1 bis 10, wobei die doppelsträngige Nucleinsäuregruppierung einen Nick in einem oder beiden Nucleinsäuresträngen aufweist.

12. Nucleinsäuremolekül nach einem der Ansprüche 1 bis 11, wobei die doppelsträngige Nucleinsäuregruppierung einen Triplex oder Quadruplex aufweist.

13. Nucleinsäuremolekül nach einem der Ansprüche 1 bis 12, das nur aus DNA besteht.

14. Pharmazeutische Zusammensetzung, die ein Nucleinsäuremolekül nach einem der Ansprüche 1 bis 13 als Wirkstoff umfasst.

## Revendications

1. Molécule d'acide nucléique pour inhiber une activité d'une molécule d'ARNi cible, comprenant :
un fragment d'acide nucléique simple brin comprenant une ou plusieurs régions d'ADN non modifiées consistant en une séquence de nucléotides suffisamment complémentaire à une séquence de nucléotides d'un brin fonctionnel possédant l'activité dans la molécule d'ARNi cible ; et
un fragment d'acide nucléique double brin lié à au moins une de l'extrémité 5' et l'extrémité 3' du fragment d'acide nucléique simple brin,
dans laquelle au moins une région d'ADN non modifiée comprend un site de mésappariement de 3 à 8 nucléotides successifs qui forme une boucle lorsque l'on permet à la région d'ADN non modifiée de former des paires de bases avec le brin fonctionnel.

2. Molécule d'acide nucléique selon la revendication 1, dans laquelle au moins une région d'ADN non modifiée possède une longueur de 18 à 35 nucléotides.

3. Molécule d'acide nucléique selon la revendication 1 ou 2, dans laquelle le fragment d'acide nucléique simple brin comprend deux régions d'ADN non modifiées ou plus qui sont identiques ou différentes.

4. Molécule d'acide nucléique selon la revendication 3, comprenant en outre une région espaceur consistant en un acide nucléique possédant une longueur de 1 à 10 nucléotides entre deux des régions d'ADN non modifiées et qui relie les régions ensemble.

5. Molécule d'acide nucléique selon l'une quelconque des revendications 1 à 4, comprenant deux fragments d'acide nucléique simple brin ou plus qui sont identiques ou différents.

6. Molécule d'acide nucléique selon l'une quelconque des revendications 1 à 5, dans laquelle le fragment ou les fragments d'acide nucléique simple brin comprennent une région de liaison consistant en un acide nucléique possédant une longueur de 1 à 10 nucléotides afin de médier la liaison entre une des régions d'ADN non modifiées et le fragment d'acide nucléique double brin.

7. Molécule d'acide nucléique selon l'une quelconque des revendications 1 à 6, dans laquelle la molécule d'ARNi est un ARNsi, un ARNsh, ou un ARNmi.

8. Molécule d'acide nucléique selon l'une quelconque des revendications 1 à 7, dans laquelle le fragment d'acide nucléique double brin possède une longueur de 5 à 25 nucléotides.

9. Molécule d'acide nucléique selon l'une quelconque des revendications 1 à 8, dans laquelle le fragment d'acide nucléique double brin comporte un site de mésappariement d'un seul nucléotide ou de 2 à 6 nucléotides successifs.

10. Molécule d'acide nucléique selon l'une quelconque des revendications 1 à 9, dans laquelle le fragment d'acide nucléique double brin comporte une région de boucle consistant en un acide nucléique possédant une longueur de 3 à 10 nucléotides et reliant l'extrémité 3' d'un brin d'acide nucléique du fragment d'acide nucléique double brin et l'extrémité 5' de l'autre brin d'acide nucléique.

11. Molécule d'acide nucléique selon l'une quelconque des revendications 1 à 10, dans laquelle le fragment d'acide nucléique double brin comporte une coupure dans un ou les deux brins d'acide nucléique.

12. Molécule d'acide nucléique selon l'une quelconque des revendications 1 à 11, dans laquelle le fragment d'acide nucléique double brin comporte un triplex ou un quadruplex.

13. Molécule d'acide nucléique selon l'une quelconque des revendications 1 à 12, qui est composée d'ADN uniquement.

14. Composition pharmaceutique comprenant une molécule d'acide nucléique selon l'une quelconque des revendications 1 à 13 en tant qu'ingrédient actif.


### SEQUENCE LISTING

<110> Osaka City University
<120> Nucleic acid molecule for suppressing activity of RNAi molecule
<130> PH-5253-PCT
<150> JP 2011-250905
   <151> 2011-11-16
<160> 55
<170> PatentIn version 3.5
<210> 1
   <211> 22
   <212> RNA
   <213> Artificial
<220>
   <223> Synthetic RNA
<400> 1
   uagcagcacg uaaauauugg cg 22
<210> 2
   <211> 63
   <212> DNA
   <213> Artificial
<220>
   <223> Synthetic DNA
<400> 2
   gtaaaacgac ggccagtccg ccaatattta aatccgtgct gctacgccct atagtgagtc 60
   gta 63
<210> 3
   <211> 24
   <212> DNA
   <213> Artificial
<220>
   <223> Synthetic DNA
<400> 3
   cgacgttgta aaacgacggc cagt 24
<210> 4
   <211> 51
   <212> DNA
   <213> Artificial
<220>
   <223> Synthetic DNA
<400> 4
   cgccaatatt tatcgacgtg ctgctacact ggccgtcgtt ttacaacgtc g 51
<210> 5
   <211> 44
   <212> DNA
   <213> Artificial
<220>
   <223> Synthetic DNA
<400> 5
   gtaaaacgac ggccagtccg ccaatattta aatccgtgct gcta 44
<210> 6
   <211> 17
   <212> DNA
   <213> Artificial
<220>
   <223> Synthetic DNA
<400> 6
   actggccgtc gttttac 17
<210> 7
   <211> 63
   <212> DNA
   <213> Artificial
<220>
   <223> Synthetic DNA
<400> 7
   tacgactcac tatagggccc gccaatattt atcgacgtgc tgctacactg gccgtcgttt 60
   tac 63
<210> 8
   <211> 18
   <212> DNA
   <213> Artificial
<220>
   <223> Synthetic DNA
<400> 8
   gccctatagt gagtcgta 18
<210> 9
   <211> 70
   <212> DNA
   <213> Artificial
<220>
   <223> Synthetic DNA
<400> 9
   tacgactcac tatagggccc gccaatattt atcgacgtgc tgctacactg gccgtcgttt 60
   tacaacgtcg 70
<210> 10
   <211> 51
   <212> DNA
   <213> Artificial
<220>
   <223> Synthetic DNA
<400> 10
   cgccaatatt taaatccgtg ctgctaccga cgttgtaaaa cgacggccag t 51
<210> 11
   <211> 44
   <212> DNA
   <213> Artificial
<220>
   <223> Synthetic DNA
<400> 11
   actggccgtc gttttacccg ccaatattta tcgacgtgct gcta 44
<210> 12
   <211> 22
   <212> DNA
   <213> Artificial
<220>
   <223> Synthetic DNA
<400> 12
   gtaatacgac tcactatagg gc 22
<210> 13
   <211> 63
   <212> DNA
   <213> Artificial
<220>
   <223> Synthetic DNA
<400> 13
   tacgactcac tatagggctt caacatcagt ctagatgata agctatactg gccgtcgttt 60
   tac 63
<210> 14
   <211> 22
   <212> RNA
   <213> Artificial
<220>
   <223> Synthetic RNA
<400> 14
   uagcuuauca gacugauguu ga 22
<210> 15
   <211> 64
   <212> DNA
   <213> Artificial
<220>
   <223> Synthetic DNA
<400> 15
   tacgactcac tatagggctc tactaaaaca tgtcgagaag cacttatact ggccgtcgtt 60
   ttac 64
<210> 16
   <211> 23
   <212> RNA
   <213> Artificial
<220>
   <223> Synthetic RNA
<400> 16
   uaagugcuuc cauguuuuag uag 23
<210> 17
   <211> 62
   <212> DNA
   <213> Artificial
<220>
   <223> Synthetic DNA
<400> 17
   gcggttggtg tggttgggcc cgccaatatt tacgtgctgc tacgcggttg gtgtggttgg 60
   gc 62
<210> 18
   <211> 63
   <212> DNA
   <213> Artificial
<220>
   <223> Synthetic DNA
<400> 18
   gcggttggtg tggttgggcc cgccaatatt tatcgtgctg ctacgcggtt ggtgtggttg 60
   ggc 63
<210> 19
   <211> 64
   <212> DNA
   <213> Artificial
<220>
   <223> Synthetic DNA
<400> 19
   gcggttggtg tggttgggcc cgccaatatt tatacgtgct gctacgcggt tggtgtggtt 60
   gggc 64
<210> 20
   <211> 65
   <212> DNA
   <213> Artificial
<220>
   <223> Synthetic DNA
<400> 20
   gcggttggtg tggttgggcc cgccaatatt tatcacgtgc tgctacgcgg ttggtgtggt 60
   tgggc 65
<210> 21
   <211> 67
   <212> DNA
   <213> Artificial
<220>
   <223> Synthetic DNA
<400> 21
   gcggttggtg tggttgggcc cgccaatatt tatctgacgt gctgctccgc ggttggtgtg 60
   gttgggc 67
<210> 22
   <211> 68
   <212> DNA
   <213> Artificial
<220>
   <223> Synthetic DNA
<400> 22
   gcggttggtg tggttgggcc cgccaatatt tatctagacg tgctgctccg cggttggtgt 60
   ggttgggc 68
<210> 23
   <211> 63
   <212> DNA
   <213> Artificial
<220>
   <223> Synthetic DNA
<400> 23
   gtaaaacgac ggccagtccg cccgggaata tttacgtgct gctacgccct atagtgagtc 60
   gta 63
<210> 24
   <211> 63
   <212> DNA
   <213> Artificial
<220>
   <223> Synthetic DNA
<400> 24
   tacgactcac tatagggccc gcctcgaaat atttacgtgc tgctacactg gccgtcgttt 60
   tac 63
<210> 25
   <211> 63
   <212> DNA
   <213> Artificial
<220>
   <223> Synthetic DNA
<400> 25
   gtaaaacgac ggccagtccg ccaatattta cgtgctgcag gctacgccct atagtgagtc 60
   gta 63
<210> 26
   <211> 63
   <212> DNA
   <213> Artificial
<220>
   <223> Synthetic DNA
<400> 26
   tacgactcac tatagggccc gccaatattt acgtgctcga ggctacactg gccgtcgttt 60
   tac 63
<210> 27
   <211> 21
   <212> RNA
   <213> Artificial
<220>
   <223> Synthetic RNA
<400> 27
   ugagaugaag cacuguagcu c 21
<210> 28
   <211> 62
   <212> DNA
   <213> Artificial
<220>
   <223> Synthetic DNA
<400> 28
   gtaaaacgac ggccagttga gctacagtgc aagcttcatc tcatgcccta tagtgagtcg 60
   ta 62
<210> 29
   <211> 62
   <212> DNA
   <213> Artificial
<220>
   <223> Synthetic DNA
<400> 29
   tacgactcac tatagggctg agctacagtg ctcgattcat ctcatactgg ccgtcgtttt 60
   ac 62
<210> 30
   <211> 63
   <212> DNA
   <213> Artificial
<220>
   <223> Synthetic DNA
<400> 30
   gtaaaacgac ggccagtttc aacatcagtc gacttgataa gctatgccct atagtgagtc 60
   gta 63
<210> 31
   <211> 64
   <212> DNA
   <213> Artificial
<220>
   <223> Synthetic DNA
<400> 31
   gcggttggtg tggttgggcc gccaatattt atcgacgtgc tgctagcggt tggtgtggtt 60
   gggc 64
<210> 32
   <211> 66
   <212> DNA
   <213> Artificial
<220>
   <223> Synthetic DNA
<400> 32
   gcggttggtg tggttgggcc cgccaatatt tatcgacgtg ctgctacgcg gttggtgtgg 60
   ttgggc 66
<210> 33
   <211> 68
   <212> DNA
   <213> Artificial
<220>
   <223> Synthetic DNA
<400> 33
   gcggttggtg tggttgggct tcgccaatat ttatcgacgt gctgctattg cggttggtgt 60
   ggttgggc 68
<210> 34
   <211> 70
   <212> DNA
   <213> Artificial
<220>
   <223> Synthetic DNA
<400> 34
   gcggttggtg tggttgggct ttcgccaata tttatcgacg tgctgctatt tgcggttggt 60
   gtggttgggc 70
<210> 35
   <211> 72
   <212> DNA
   <213> Artificial
<220>
   <223> Synthetic DNA
<400> 35
   gcggttggtg tggttgggct tttcgccaat atttatcgac gtgctgctat tttgcggttg 60
   gtgtggttgg gc 72
<210> 36
   <211> 74
   <212> DNA
   <213> Artificial
<220>
   <223> Synthetic DNA
<400> 36
   gcggttggtg tggttgggct ttttcgccaa tatttatcga cgtgctgcta tttttgcggt 60
   tggtgtggtt gggc 74
<210> 37
   <211> 30
   <212> DNA
   <213> Artificial
<220>
   <223> Synthetic DNA
<400> 37
   tttttttttc aatacgactc actatagggc 30
<210> 38
   <211> 59
   <212> DNA
   <213> Artificial
<220>
   <223> Synthetic DNA
<400> 38
   cggccagcgc caatatttac gtgctgctag ccctatagtg agtcgtatta cggatcccg 59
<210> 39
   <211> 59
   <212> DNA
   <213> Artificial
<220>
   <223> Synthetic DNA
<400> 39
   cggccagcgc caatatttac gtgctgctag ccctatagtg agtcgtatta cggatcccg 59
<210> 40
   <211> 69
   <212> DNA
   <213> Artificial
<220>
   <223> Synthetic DNA
<400> 40
   ctggccggaa cggccagcgc caatatttac gtgctgctag ccctatagtg agtcgtatta 60
   cggatcccg 69
<210> 41
   <211> 15
   <212> DNA
   <213> Artificial
<220>
   <223> Synthetic DNA
<400> 41
   ggttggtgtg gttgg 15
<210> 42
   <211> 57
   <212> DNA
   <213> Artificial
<220>
   <223> Synthetic DNA
<400> 42
   agatctcgag aagcttagat atcgtcgacc cgggatccac cggatctaga taactga 57
<210> 43
   <211> 70
   <212> DNA
   <213> Artificial
<220>
   <223> Synthetic DNA
<400> 43
   gtagcgccaa tatttacgtg ctgctacgcc aatatttacg tgctgctacg ccaatattta 60
   cgtgctgcta 70
<210> 44
   <211> 70
   <212> DNA
   <213> Artificial
<220>
   <223> Synthetic DNA
<400> 44
   tagcagcacg taaatattgg cgtagcagca cgtaaatatt ggcgtagcag cacgtaaata 60
   ttggcgctac 70
<210> 45
   <211> 79
   <212> DNA
   <213> Artificial
<220>
   <223> Synthetic DNA
<400> 45
   gtagcaacgt tgaggaaggt gactgccaac aacgttgagg aaggtgactg ccaacaacgt 60
   tgaggaaggt gactgccaa 79
<210> 46
   <211> 79
   <212> DNA
   <213> Artificial
<220>
   <223> Synthetic DNA
<400> 46
   ttggcagtca ccttcctcaa cgttgttggc agtcaccttc ctcaacgttg ttggcagtca 60
   ccttcctcaa cgttgctac 79
<210> 47
   <211> 64
   <212> DNA
   <213> Artificial
<220>
   <223> Synthetic DNA
<400> 47
   gtaaaacgac ggccagtccg cccaatattt aaatccgtgc tgctacgccc tatagtgagt 60
   cgta 64
<210> 48
   <211> 71
   <212> DNA
   <213> Artificial
<220>
   <223> Synthetic DNA
<400> 48
   tacgactcac tatagggccc gcccaatatt tatcgacgtg ctgctacact ggccgtcgtt 60
   ttacaacgtc g 71
<210> 49
   <211> 22
   <212> DNA
   <213> Artificial
<220>
   <223> Synthetic DNA
<220>
   <221> misc_feature
   <222> (1)..(1)
   <223> BNA
<220>
   <221> misc_feature
   <222> (5)..(5)
   <223> BNA
<220>
   <221> misc-feature
   <222> (8)..(8)
   <223> BNA
<220>
   <221> misc_feature
   <222> (11)..(11)
   <223> BNA
<220>
   <221> misc_feature
   <222> (15)..(15)
   <223> BNA
<220>
   <221> misc_feature
   <222> (18)..(18)
   <223> BNA
<220>
   <221> misc_feature
   <222> (21)..(22)
   <223> BNA
<400> 49
   cgccaatatt tacgtgctgc ta 22
<210> 50
   <211> 22
   <212> RNA
   <213> Artificial
<220>
   <223> Modified by 2'-OMe
<400> 50
   cgccaauauu uacgugcugc ua 22
<210> 51
   <211> 65
   <212> DNA
   <213> Artificial
<220>
   <223> Synthetic DNA
<400> 51
   gtcgacgcta gcctgcgtcc actgaaacat gtctagaagc acttatgtaa tcatggtcat 60
   agctg 65
<210> 52
   <211> 65
   <212> DNA
   <213> Artificial
<220>
   <223> Synthetic DNA
<400> 52
   cagctatgac catgattact acactcaaac atgtcgagaa gcacttatcg caggctagcg 60
   tcgac 65
<210> 53
   <211> 60
   <212> DNA
   <213> Artificial
<220>
   <223> Synthetic DNA
<400> 53
   ggcccgaagg gcctttcaac atcagtctcg atgataagct agttgaaccg gcgaagccgg 60
<210> 54
   <211> 60
   <212> DNA
   <213> Artificial
<220>
   <223> Synthetic DNA
<400> 54
   ggaccgaagg tcctttcaca agttagtcga ggtctcaggg agtgaaagaa cggaacgttc 60
<210> 55
   <211> 60
   <212> DNA
   <213> Artificial
<220>
   <223> Synthetic DNA
<400> 55
   ggaccgaagg tccttcaagc tgattttcga acacccggtg acttgaagtc ccgaagggac 60